(19) Europäisches Patentamt
European Patent Office
Office européen des brevets

(11) EP 0 671 392 B1

(12) EUROPÄISCHE PATENTSCHRIFT

(45) Veröffentlichungstag und Bekanntmachung des Hinweises auf die Patenterteilung:
**21.02.2001 Patentblatt 2001/08**

(51) Int Cl.[7]: **C07D 235/06**, C07D 471/04, A61K 31/415

(21) Anmeldenummer: **95103046.9**

(22) Anmeldetag: **03.03.1995**

(54) **Substituierte Propan-Derivate, Verfahren zu ihrer Herstellung und die Anwendung der Verbindungen zur Behandlung von Krankheiten**

Substituted propane derivatives, process for their preparation and use of the compounds for the treatment of illnesses

Dérivés de propane substitués, procédé pour leur préparation et leur utilisation pour le traitement de maladies

(84) Benannte Vertragsstaaten:
**AT BE CH DE DK ES FR GB GR IE IT LI LU NL PT SE**

(30) Priorität: **10.03.1994 DE 4408082**

(43) Veröffentlichungstag der Anmeldung:
**13.09.1995 Patentblatt 1995/37**

(73) Patentinhaber: **HOECHST AKTIENGESELLSCHAFT**
**65929 Frankfurt am Main (DE)**

(72) Erfinder:
- **Schubert, Gerrit, Dr.**
  **D-65779 Kelkheim (DE)**
- **Hemmerle, Horst, Dr.**
  **D-64653 Lorsch (DE)**
- **Below, Peter, Dr.**
  **D-60529 Frankfurt (DE)**
- **Herling, Andreas, Dr.**
  **D-65520 Bad Camberg (DE)**
- **Burger, Hans-Jörg, Dr.**
  **D-65719 Hofheim (DE)**

(56) Entgegenhaltungen:
**EP-A- 0 587 087** **EP-A- 0 587 088**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Das Krankheitsbild des Diabetes ist durch erhöhte Blutzuckerwerte gekennzeichnet. Beim insulinpflichtigen oder Typ I Diabetes ist die Ursache das Absterben der insulinproduzierenden β-Zellen des Pankreas; die Behandlung erfolgt daher durch Insulingabe (Substitutionstherapie). Der nicht-insulinabhängige oder Typ II Diabetes ist dagegen durch eine verminderte Insulinwirkung auf Muskel- und Fettgewebe (Insulinresistenz) und eine gesteigerte Glucoseproduktion der Leber gekennzeichnet. Die Ursachen dieser Stoffwechselstörungen sind noch weitgehend ungeklärt. Die etablierte Therapie mit Sulfonylharnstoffen versucht die Insulinresistenz durch Steigerung der körpereigenen Insulinfreisetzung zu kompensieren, führt jedoch nicht in allen Fällen zu einer Normalisierung des Blutzuckerspiegels und vermag das Fortschreiten der Krankheit nicht aufzuhalten; viele Typ II Diabetiker werden schließlich durch "Erschöpfung" der β-Zellen insulinpflichtig und leiden an Spätschäden wie Katarakten, Nephropathien und Angiopathien. Neue Therapieprinzipien zur Behandlung des Typ II Diabetes sind deshalb wünschenswert.
Die Konzentration der Blutglucose im nüchternen Zustand wird durch die Glucoseproduktion der Leber bestimmt. Verschiedene Arbeitsgruppen konnten zeigen, daß die Erhöhung der Blutzuckerwerte bei Typ II Diabetes mit einer proportional erhöhten Glucoseabgabe aus der Leber korreliert. Die von der Leber in das Blut abgegebene Glucose kann sowohl durch Abbau von Leber-Glycogen (Glycogenolyse) als auch durch Gluconeogenese gebildet werden.
Glucose-6-Phosphat ist das gemeinsame Endprodukt sowohl der Gluconeogenese als auch der Glykogenolyse. Der terminale Schritt der hepatischen Freisetzung von Glucose aus Glucose-6-Phosphat wird von der Glucose-6-Phosphatase (EC 3.1.3.9) katalysiert. Die Glucose-6-Phosphatase stellt einen im endoplasmatischen Reticulum (ER) vorkommenden Multienzym-Komplex dar. Dieser Enzym-Komplex besteht aus einer in der ER-Membran vorliegenden Glucose-6-Phosphat-Translocase, einer auf der luminalen Seite des endoplasmatischen Reticulum lokalisierten Glucose-6-Phosphatase und einer Phosphat-Translocase [für eine Übersicht siehe: Ashmore, J. und Weber G., "The Role of Hepatic Glucose-6-phosphatase in the Regulation of Carbohydrate Metabolism", in Vitamins and Hormones, Vol XVII (Harris R.S., Marrian G.F., Thimann K.V., Edts.), 92-132, (1959); Burchell A., Waddell I.D., "The molecular basis of the hepatic microsomal glucose-6-phosphatase system", Biochim. Biophys. Acta 1092, 129-137, (1990)]. Die vorliegende umfangreiche Literatur zeigt, daß unter allen untersuchten Bedingungen, die im Tierversuch zu erhöhten Blutglucose-Werten führen, z.B. Streptozotocin, Alloxan, Cortison, Thyroid-Hormone und Hungern, die Aktivität dieses Multienzym-Komplexes ebenfalls erhöht ist. Darüber hinaus weisen zahlreiche Untersuchungen darauf hin, daß die bei Typ II Diabetikern beobachtete erhöhte Glucose-Produktion mit einer erhöhten Glucose-6-Phosphatase-Aktivität verbunden ist. Die Bedeutung des Glucose-6-Phosphatase-Systems für eine normale Glucose-Homeostase wird ferner unterstrichen durch die hypoglykämischen Symptome von Patienten mit Glykogen-Speicherkrankheit Typ Ib, denen die Translocase-Komponente des Glucose-6-Phosphatase-Systems fehlt.
Eine Verringerung der Glucose-6-Phosphatase-Aktivität durch geeignete Wirkstoffe (Inhibitoren) sollte zu einer entsprechend verringerten hepatischen Glucose-Freisetzung führen. Diese Wirkstoffe sollten in der Lage sein, die Glucose-Produktion der Leber dem effektiven peripheren Verbrauch anzupassen. Die dadurch im nüchternen Zustand von Typ II Diabetikern gesenkten Blutglucose-Werte dürften darüber hinaus auch eine präventive Wirkung im Hinblick auf diabetische Spätschäden haben.
In der Literatur ist eine Reihe von unspezifischen Inhibitoren der Glucose-6-Phosphatase beschrieben worden wie z. B. Phlorrhizin [Soodsma, J. F., Legler, B. und Nordlie, R. C., J. Biol. Cherr. 242, 1955-1960, (1967)], 5,5'-Dithio-bis-2-nitrobenzoesäure [Wallin, B. K. und Arion, W. J., Biochem. Biophys. Res. Commun. 48, 694-699, (1972)], 2,2'-Di-isothiocyanato-stilben und 2-Isothiocyanato-2'-acetoxy-stilben [Zoccoli, M. A. und Karnowski, M. L., J. Biol. Chem. 255, 1113-1119, (1980)]. Die ersten therapeutisch verwertbaren Inhibitoren des Glucose-6-Phosphatase-Systems werden in den europäischen Patentanmeldungen Nr. 93 114 260.8 und Nr. 93 114 261.6 vorgeschlagen.
Die nachfolgend näher charakterisierten Propan-Derivate sind neue, in der chemischen und biologischen Literatur bisher nicht beschriebene Verbindungen. Wir haben nun gefunden, daß bestimmte substituierte Propane wie z. B. Beispiel 1, Hemmer des Glucose-6-Phosphatase-Systems sind.

Die Erfindung betrifft daher Propan-Derivate der Formel I

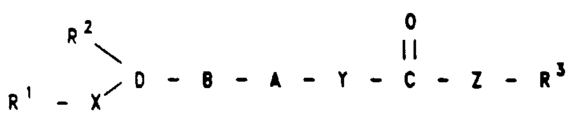

I

worin die Reste die folgenden Bedeutungen haben:

D-B-A: Propylen, das gegebenenfalls mit 1 bis 3 Gruppen ausgewählt aus $C_1$-$C_3$-Alkyl, ω-Hydroxy-($C_1$-

$C_4$)-Alkyl, OH, O-$C_1$-$C_4$-Alkyl, Phenyl und Phenyl-$C_1$-$C_3$-Alkyl substituiert ist,

$R^1$: CN, COOH, eine mit einer Schutzgruppe geschützte COOH-Gruppe, $C_1$-$C_4$-Alkanoyl, $SO_3$-$C_1$-$C_4$-Alkyl, $SO_3H$, $SO_2NR^5R^6$, $PO(OH)_2$, PO(OH)(O-$C_1$-$C_4$-Alkyl), PO(O-$C_1$-$C_4$-Alkyl$)_2$, Tetrazol-5-yl,

$R^2$: $C_1$-$C_{10}$-Alkyl $(R^8)_n$, O-$C_1$-$C_{10}$-Alkyl $(R^8)_n$,
$C_2$-$C_{10}$-Alkenyl $(R^8)_n$, O-$C_3$-$C_{10}$-Alkenyl $(R^8)_n$,
$C_2$-$C_{10}$-Alkinyl $(R^8)_n$, O-$C_3$-$C_{10}$-Alkinyl $(R^8)_n$,
S-$C_1$-$C_{10}$-Alkyl $(R^8)_n$, S-$C_3$-$C_{10}$-Alkenyl $(R^8)_n$,
S-$C_3$-$C_{10}$-Alkinyl $(R^8)_n$,
NH-$C_1$-$C_{10}$-Alkyl $(R^8)_n$, NH-$C_3$-$C_{10}$-Alkenyl $(R^8)_n$ oder
NH-$C_3$-$C_{10}$-Alkinyl $(R^8)_n$, wobei $R^8$ gegebenenfalls jeweils mit $R^9$ substituiert ist;

$R^3$, $R^8$ und $R^{10}$: Alkyl mit 1 bis 10 C-Atomen, Cycloalkyl mit 3-8 Ring-C-Atomen, Phenyl, Naphthyl, Phenanthryl, Pyridyl, Thienyl, Furyl, Pyrimidyl, Indolyl, Imidazolyl, Coumarinyl, Phthalimidyl, Chinolyl, Piperazinyl, Tetrazolyl, Triazolyl, Oxazolyl oder deren thieno-, pyridino-, pyrimidino-, pyrazino-, pyridazino- oder benzoanellierte Derivate, wobei der Aromat bzw. Heteroaromat ein- oder mehrfach mit F, Cl, Br, J, OH, $CF_3$, $-NO_2$, CN, $C_1$-$C_4$-Alkoxy, $C_1$-$C_4$-Alkyl, $NR^5R^6$, Phenyl, Benzyl, Thienyl, Furyl, Imidazolyl, Pyridyl, 0-Phenyl oder 0-Benzyl gleich oder verschieden substituiert sein kann, und $R^3$, $R^8$ und $R^{10}$ gleich oder verschieden sind;

$R^4$: $C_1$-$C_4$-Alkyl, Phenyl oder Benzyl;

$R^5$ und $R^6$: H, $C_1$-$C_4$-Alkyl, $C_1$-$C_4$-Alkanoyl, Phenyl, das gegebenenfalls substituiert ist mit F, Cl, Br, J, OH, O-$C_1$-$C_4$-Alkyl, $CF_3$, $-NO_2$ oder CN, wobei $R^5$ und $R^6$ gleich oder verschieden sind, oder $R^5$ und $R^6$ bilden zusammen mit dem Stickstoffatom einen 4 bis 10gliedrigen, gesättigten heterocyclischen Ring, bei dem gegebenenfalls eine $CH_2$-Gruppe durch O, S oder $NR^7$ ersetzt sein kann,

$R^7$: H, $C_1$-$C_4$-Alkyl, Phenyl oder Benzyl

$R^9$: Phenyl, Naphthyl, Phenanthryl, Pyridyl, Thienyl, Furyl, Pyrimidyl, Indolyl, Imidazolyl, Coumarinyl, Phthalimidyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, J, OH, $CF_3$, $-NO_2$, CN, $C_1$-$C_4$-Alkoxy, $C_1$-$C_4$-Alkyl, $C_2$-$C_4$-Alkenyl, $NR^5R^6$, Phenyl, Benzyl, Thienyl, Furyl, Imidazolyl, Pyridyl, O-Phenyl oder O-Benzyl gleich oder verschieden substituiert sein kann;

X: $(CH_2)_m$, -CH =CH-, -C≡C-, $-CH_2-O-CH_2-$, $-CH_2-S-CH_2-$ oder

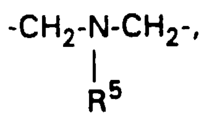

Y: $(CH_2)_m$, O, S, $NR^5$,

Z: $(CH_2)_m$, S, O, S-$C_1$-$C_{10}$-Alkyl, O-$C_1$-$C_{10}$-Alkyl, CH=CH, CH=CF, CH=CCl, CH=CBr, $CH_2$-CO, $CH_2$-CHF, $CH_2$-CHCl, $CH_2$-CHBr,
$CH_2$-CHJ, $C_3$-$C_{10}$-Cycloalkylen, $C_3$-$C_{10}$-Cycloalkenylen, wobei 1 bis 3 Ring-C-Atome durch Schwefel-, Sauerstoff- oder Stickstoffatome ersetzt sein können, $COOR^4$, C≡C, CH = C($C_1$-$C_4$-Alkyl), CH=C(CN), CH =C($NR^5R^6$), CH=C($C_1$-$C_4$-Alkanoyl), CH=C($R^{10}$), $NR^5$ und wenn Y Sauerstoff ist, kann

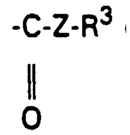

gemeinsam einen Aminosäurerest, ausgewählt aus der Gruppe bestehend aus Ala, Arg, Asn, Asp, Cys, Gln, Glu, Gly, His, Ile,Leu, Lys, Phe, Pro, Ser, Thr, Trp, Tyr und deren durch übliche Schutzgruppen geschützte Derivate, darstellen,

n: null, 1 oder 2

m: null, 1, 2, 3 oder 4.

Die erfindungsgemäßen Verbindungen der Formel I können, soweit sie eine Carboxylgruppe enthalten, Salze mit anorganischen oder organischen Basen bilden. Die Erfindung betrifft daher auch die physiologisch verträglichen Salze von Verbindungen der Formel I.
Die erfindungsgemäßen Verbindungen der Formel I enthalten eine Reihe von Stereozentren. Die Erfindung betrifft alle möglichen Enantio- und Diastereomere. Sie werden alle durch die Formel I wiedergegeben.
Sofern nichts anderes angegeben ist, gilt für die vorstehenden und folgenden Ausführungen:
Die unter $R^1$, $R^3$, $R^4$, $R^5$, $R^6$, $R^7$, $R^8$, $R^{10}$ und Z angegebenen Alkyl-, Alkanoyl und Alkoxyreste sind geradkettig oder verzweigt.
Die unter $R^2$ und $R^9$ angegebenen Alkyl-, Alkenyl- und Alkinylgruppen sind geradkettig, verzweigt oder cyclisch, wobei auch nur ein Teil des Restes einen Ring bilden kann.
Eine der $CH_2$-Gruppen kann durch O, S, SO, $SO_2$ oder $NR^5$ ersetzt sein, $R^8$ kann mit $R^9$ substituiert sein und bei n =2 sind die beiden Reste $R^8$ gleich oder verschieden. Ungesättigte Reste sind ein- oder mehrfach ungesättigt.
Unter einem mit einer Schutzgruppe geschützten COOH-Rest wird COO-$C_1$-$C_{10}$-Alkyl (unverzweigt oder verzweigt oder cyclisch), COO-CH($R^4$)-O-$C_1$-$C_4$-Alkanoyl (unverzweigt oder verzweigt), COO-Benzyl, COOPhenyl, $CONH_2$, CONH-$C_1$-$C_{10}$-Alkyl (unverzweigt und verzweigt), -$CONR^5R^6$ verstanden, wobei $R^4$, $R^5$ und $R^6$ die angegebenen Bedeutungen haben.
Alkoholschutzgruppen sind:
Substituierte Ether wie Methoxymethyl, Methylthiomethyl, t-Butylthiomethyl, Benzyloxymethyl, p-Methoxybenzyloxymethyl, t-Butoxymethyl, Siloxymethyl, 2-Methoxyethoxymethyl, 1-Ethoxyethyl, Allyl, Benzyl, p-Methoxybenzyl, 3,4-Dimethoxybenzyl, o-Nitrobenzyl, p-Nitrobenzyl, p-Halobenzyl, 2,6-Dichlorbenzyl, p-Cyanbenzyl, p-Phenylbenzyl, 2- und 4-Picolyl.
Schutzgruppen für die Aminosäuren sind:

a) Carbamate wie
Methyl und Ethyl, 9-Fluorenylmethyl, 9-(2-Sulfo)fluorenylmethyl, 9-(2,7-Dibrom)fluorenylmethyl, 2,7-Di-t-butyl-[9-(10,10-dioxo-10,10,10,10-tetrahydrothioxanthyl)]methyl, 4-Methoxyphenacyl, 2,2,2-Trichlorethyl, 2-Trimethylsilylethyl, 2-Phenylethyl, 1-(1-Adamantyl)-1-methylethyl, 1,1-Dimethyl-2-haloethyl, 1,1-Dimethyl-2,2-dibromethyl, 1,1-Dimethyl-2,2,2-trichlorethyl, 1-Methyl-1-(4-biphenylyl)ethyl, 1-(3,5-Di-t-butylphenyl)-1-methylethyl, 2-(2'- und 4'-Pyridyl)ethyl, 2-(N,N-Dicyclohexylcarboxamido)ethyl, t-Butyl, l-Adamantyl, Vinyl, Allyl, 1-Isopropylallyl, Cinnamyl, 4-Nitrocinnamyl, 8-Chinolyl, N-Hydroxypiperidinyl, Alkylthio, Benzyl, p-Methoxybenzyl, p-Nitrobenzyl, p-Brombenzyl, p-Chlorobenzyl, 2,4-Dichlorbenzyl, 4-Methylsulfinylbenzyl, 9-Anthrylmethyl und Diphenylmethyl, t-Amyl, S-Benzyl-thiocarbamat, p-Cyanobenzyl, Cyclobutyl, Cyclohexyl, Cyclopentyl, Cyclopropylmethyl, p-Decyloxybenzyl, Diisopropylmethyl, 2,2-Dimethoxycarbonylvinyl, o-(N,N-Dimethylcarboxamido)benzyl, 1,1-Dimethyl-3-(N,N-dimethylcarboxamido)propyl, 1,1-Dimethylpropynyl, Di-(2-pyridyl)methyl, 2-Furanylmethyl, 2-Jodoethyl, Isobornyl, Isobutyl, Isonicotinyl, p-(p'-Methoxyphenylazo)benzyl, 1-Methylcyclobutyl, 1-Methylcyclohexyl, 1-Methyl-1-cyclopropylmethyl, 1-Methyl-1-(3,5-dimethoxyphenyl)ethyl, 1-Methyl-1-(p-phenylazophenyl)ethyl, 1-Methyl-1-phenylethyl, 1-Methyl-1-(4-pyridyl)ethyl, Phenyl, p-(Phenylazo)benzyl, 2,4,6-Tri-t-butylphenyl, 4-(Trimethylammonium)benzyl und 2,4,6-Trimethylbenzyl.

b) Harnstoffderivate wie
Phenothiazinyl-(10)-carbonyl Derivate, N'-p-Toluolsulfonylaminocarbonyl und N'-Phenylaminothiocarbonyl.

c) Amide wie

N-Formyl, N-Acetyl, N-Chloroacetyl, N-Trichloroacetyl, N-Trifluoracetyl, N-Phenylacetyl, N-3-Phenylpropionyl, N-Picolinoyl, N-3-Pyridylcarboxamid, N-Benzoylphenylalanyl Derivate, N-Benzoyl und N-p-Phenylbenzoyl.

Bevorzugt sind Verbindungen der Formel I, in denen

$R^1$ CN, COOH, eine mit einer Schutzgruppe geschützte COOH-Gruppe oder $C_1$-$C_4$-Alkanoyl bedeutet und die übrigen Reste die vorstehenden Bedeutungen haben. Besonders bevorzugt sind Verbindungen der Formel I, worin die Reste folgende Bedeutungen haben:

$R^1$: CN, COOH, eine mit einer Schutzgruppe geschützte COOH-Gruppe oder $C_1$-$C_4$-Alkanoyl oder Tetrazol-5-yl,

$R^2$: O-$C_1$-$C_{10}$-Alkyl$(R^8)_n$ (n=0,1,2), wobei der Alkylteil unverzweigt, verzweigt oder cyclisch ist, sowie eine der $CH_2$-Gruppen durch O ersetzt sein kann und $R^8$ mit $R^9$ substituiert sein kann und bei n = 2 die beiden Reste $R^8$ gleich oder verschieden sind,
O-$C_3$-$C_{10}$-Alkenyl$(R^8)_n$ (n=0,1,2), wobei der Alkenylteil unverzweigt, verzweigt oder cyclisch ist, eine der $CH_2$-Gruppen durch O, S, SO, $SO_2$, oder $NR^5$ ersetzt sein kann sowie ein- oder mehrfach ungesättigt ist, und $R^8$ mit $R^9$ substituiert sein kann und bei n=2 die beiden Reste $R^8$ gleich oder verschieden sind,
O-$C_3$-$C_{10}$-Alkinyl$(R^8)_n$ (n =0,1,2), wobei der Alkinylteil unverzweigt, verzweigt oder cyclisch ist, ein- oder mehrfach ungesättigt ist sowie eine der $CH_2$-Gruppen durch O, S, SO, $SO_2$, oder $NR^5$ ersetzt sein kann und $R^8$ mit $R^9$ substituiert sein kann und bei n=2 die beiden Reste $R^8$ gleich oder verschieden sind,

$R^3$ bis $R^{10}$ die vorstehend angegebenen Bedeutungen haben,

X: $(CH_2)_m$, (m=0,1,2,3,4) CH=CH, C≡C, $CH_2OCH_2$, $CH_2SCH_2$

Y: $(CH_2)_m$, (m=0,1,2,3,4) 0, S, $NR^5$,

Z: $(CH_2)_m$, (m=0,1,2,3,4) S, 0, S-$C_1$-$C_{10}$-Alkyl, (unverzweigt oder verzweigt), CH=CH, CH=CF, CH=CCl, CH=CBr, $CH_2$-C(O), $CH_2$-CHF, $CH_2$-CHCl, $CH_2$-CHBr, $CH_2$-CHJ, $C_3$-$C_{10}$-Cycloalkylen, $C_3$-$C_{10}$-Cycloalkenylen, $COOR^4$, C≡C, CH=C($C_1$-$C_4$-Alkyl) (unverzweigt oder verzweigt), CH=C(CN), CH=C($R^{10}$), $NR^5$. Der Propylrest D-B-A ist vorzugsweise unsubstituiert oder an B mit 2 Methylgruppen substituiert.

Die erfindungsgmäßen Verbindungen der Formel I können, soweit sie eine Carboxylgruppe enthalten, Salze mit anorganischen oder organischen Basen bilden. Bevorzugt sind Salze mit anorganischen Basen, besonders die physiologisch unbedenklichen Alkalimetall-Salze, vor allem Natrium- und Kaliumsalze.

Die Verbindungen der Formel I hemmen das Glucose-6-Phosphatase-System der Leber in Säugetieren. Die Verbindungen eignen sich daher als Arzneimittel. Die Erfindung betrifft daher auch Arzneimittel auf Basis der Verbindungen der Formel, gegebenenfalls in Form der physiologisch verträglichen Salze.

Die Erfindung beinhaltet weiterhin die Anwendung von Verbindungen der Formel I bzw. der Salze zur Herstellung von Arzneimitteln zur Behandlung des Typ II Diabetes und anderer Erkrankungen, die durch einen erhöhten Glucose-Ausstoß aus der Leber oder eine erhöhte Aktivität des Glucose-6-Phosphatase-Systems gekennzeichnet sind.

Die Wirkung der erfindungsgemäßen Verbindungen auf das Glucose-6-Phosphatase-System wurde in einem Enzymtest in Lebermikrosomen untersucht.

Für die Präparation der die Glucose-6-Phosphatase enthaltenden Mikrosomen-Fraktion wurden frische Leber-Organe von männlichen Wistar-Ratten verwendet und wie in der Literatur beschrieben aufgearbeitet [Canfield, W. K. und Arion, W. J., J. Biol. Chem. 263, 7458-7460, (1988)]. Diese Mikrosomen-Fraktion kann bei -70 °C mindestens 2 Monate lang ohne signifikanten Aktivitätsverlust aufbewahrt werden.

Der Nachweis der Glucose-6-Phosphatase-Aktivität wurde wie in der Literatur angegeben (Arion, W. J. in Methods Enzymol. 174, Academic Press 1989, Seite 58-67) durch Bestimmung des aus Glucose-6-Phosphat freigesetzten Phosphats durchgeführt. 0,1 ml Testansatz enthielten Glucose-6-Phosphat (1 mMol/l), die Testsubstanz, 0,1 mg Mikrosomen-Fraktion und 100 mMol/l HEPES-Puffer (4-(2-Hydroxyethyl)piperazin-1-ethansulfonsäure), pH 7,0. Die Reaktion wurde durch Zugabe des Enzyms gestartet. Nach Ablauf von 20 min bei Raumtemperatur wurde die Reaktion durch Zugabe von 0,2 ml Phosphat-Reagens gestoppt. Die Probe wurde 30 min lang bei 37 °C inkubiert, und die Absorption (A) der blauen Farbe anschließend bei 570 nm gemessen. Die inhibitorische Wirksamkeit der Testsubstanz ergab sich durch Vergleich mit einer Kontroll-Reaktion, die keine Testsubstanz enthielt nach der Formel

$$\text{Prozent Hemmung} = \frac{\text{A(Kontrolle) - A(Testsubstanz)}}{\text{A(Kontrolle)}} \text{X } 100$$

Sofern erforderlich wurde die hemmende Wirkung der Testsubstanz als Funktion der eingesetzten Konzentration der Testsubstanz ermittelt, und daraus die Konzentration für die 50 %ige Hemmung der Enzymaktivität ($IC_{50}$) berechnet.

Für die nachfolgend aufgeführte Verbindung wurde der $IC_{50}$-Wert ermittelt: Verbindung aus Beispiel 1:

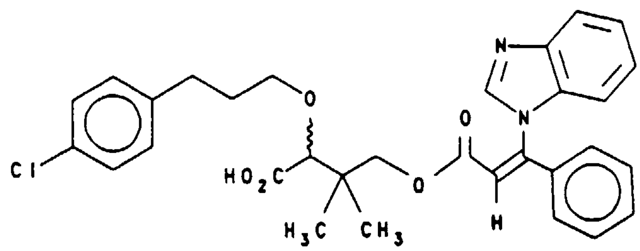

(±)-4-(3Z-Benzimidazol-1-yl-3-phenyl-propenoyloxy)-2-(4-chlorphenylpropyloxy)-3,3-dimethylbutansäure
$IC_{50}$ = 4µM

Die Herstellung der erfindungsgemäßen Verbindungen der Formel I, in denen die Reste $R^2$ = O-Alkyl$(R^8)_n$, O-Alkenyl$(R^8)_n$ oder O-Alkinyl$(R^8)_n$ und Y = O ist, kann auf den im folgenden Schema aufgezeigten Weg A erfolgen.

Verfahren A

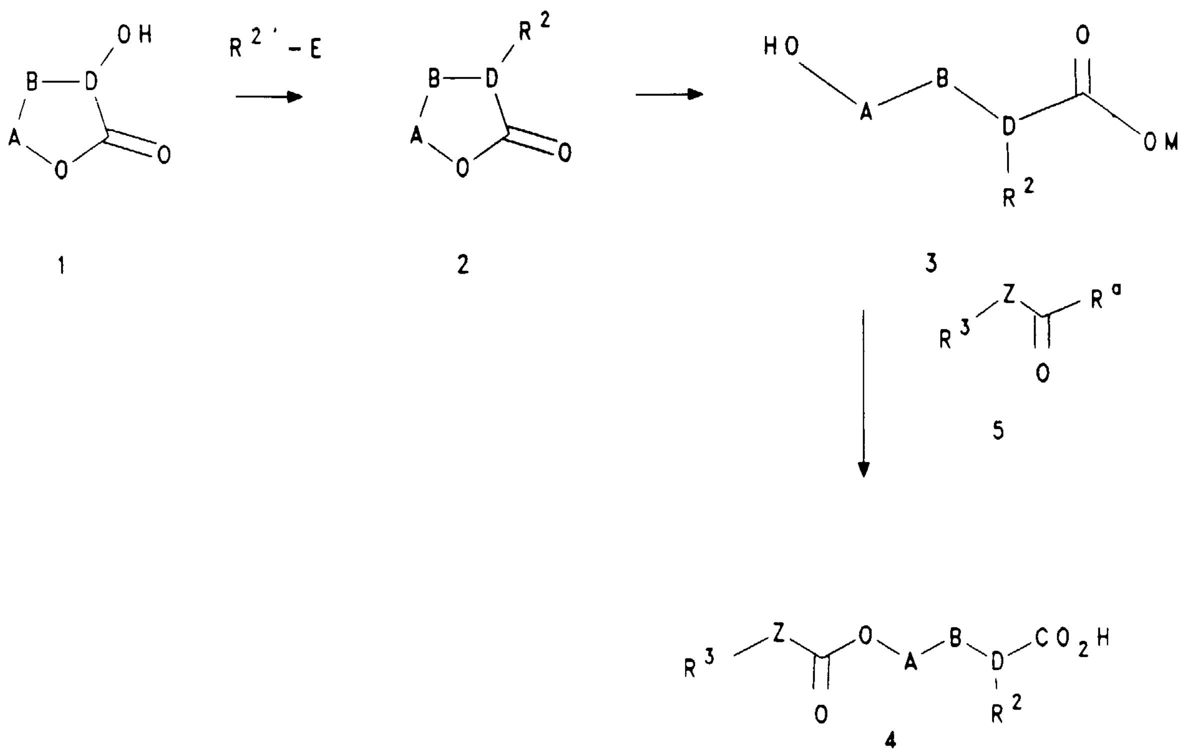


1 2 3 5 4

M: Alkalimetall
$R^a$: Cl, Br,

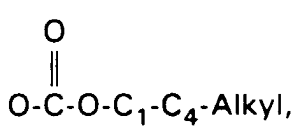

Imidazolyl, Triazolyl oder Tetrazolyl
E: Chlor, Brom, Jod, Sulfonsäureester
$R^{2'}$: Alkyl$(R^8)_n$, Alkenyl$(R^8)_n$ oder Alkinyl$(R^8)_n$

(4 = Formel I, $R^2$ = O-$C_1$-$C_{10}$-Alkyl($R^8$)$_n$, O-$C_3$-$C_{10}$-Alkenyl($R^8$)$_n$ oder O-$C_3$-$C_{10}$-Alkinyl($R^8$)$_n$, Y = O, X = $(CH_2)_m$ mit m = null, $R^1$ = COOH, Z, $R^3$, $R^8$ und n wie zu Formel I angegeben).

Die Ausgangsverbindung 1 ist bekannt oder wird nach literaturbekannten Verfahren hergestellt. Die Verbindung 1 wird mit einer starken Base wie Kalium-tert.-butylat, Natriumhydrid oder Kaliumhydrid deprotoniert und zur Einführung von $R^2$ mit entsprechenden Halogeniden, Trifluorsulfonsäureestern, Methylsulfonsäureestern oder p-Toluolsulfonsäureestern vorteilhafterweise in polaren aprotischen Lösungsmittel wie Dimethylformamid, Dimethylsulfoxid oder Tetrahydrofuran umgesetzt, wobei Verbindung 2 entsteht. Bevorzugt wird die Verwendung von Natriumhydrid als Base und Dimethylformamid als Lösungsmittel.

Die Reaktion von 1 zu 2 wird bei Temperaturen von -20°C bis zum Siedepunkt des verwendeten Lösungsmittels durchgeführt. Bevorzugt ist ein Temperaturbereich von -10 bis 60°C, besonders von 0 bis 30°C.

Die bevorzugte Ausführungsform der Reaktion 1 zu 2 erfolgt in Dimethylformamid in Gegenwart von Natriumhydrid oder Kaliumhydrid bei Temperaturen von 0 bis 60°C. Die Reaktion wird dabei vorteilhafterweise unter Feuchtigkeitsausschluß unter einem Schutzgas (Stickstoff oder Argon) durchgeführt.

Die für die Umsetzung von 1 nach 2 notwendigen Ausgangsmaterialien, die dem Rest $R^2$ entsprechen, lassen sich nach dem für den Fachmann bekannten Standardverfahren darstellen. Hierbei handelt es sich um Strukturen des Typs $R^{2'}$-E mit der für Verfahren A genannten Einschränkung (allerdings ohne das verknüpfende Sauerstoffatom). E hat z. B. die Bedeutung einer Abgangsgruppe wie Cl, Br, J, oder $OSO_2R$ (R = $CH_3$, Ph, Tolyl, $CF_3$).

Weiterer Schritt bei Verfahren A ist die Hydrolyse des Lactons 2 zum Alkalisalz 3 mit Alkalihydroxiden wie Lithiumhydroxid, Natriumhydroxid oder Kaliumhydroxid. Die Reaktion erfolgt vorteilhafterweise in protischen oder aprotischen Lösungsmitteln wie niederen Alkoholen, Tetrahydrofuran oder Dioxan, bevorzugt ist die Verwendung von Dioxan.

Die Reaktion von 2 zu 3 wird bei Temperaturen von -20°C bis zum Siedepunkt des verwendeten Lösungsmittels durchgeführt. Bevorzugt ist ein Temperaturbereich von -10 bis 60°C, besonders von 0 bis 30°C.

Weiterer Schritt ist die Reaktion von 3 zu 4, bei der der Rest $R^3$-Z-C(O)- an 3 angebunden wird. Dazu wird 3 in einem aprotischen organischen Lösungsmittel wie z.B. Tetrahydrofuran, Dimethylformamid, Dichlormethan, Pyridin oder Dimethylsulfoxid mit einer Verbindung $R^3$-Z-C(O)-$R^a$ (5) umgesetzt, wobei $R^a$ z.B. Cl, Br, OC(O)-$C_1$-$C_4$-Alkyl, Imidazolyl, Triazolyl oder Tetrazolyl sein kann, wobei Imidazolyl und Triazolyl besonders bevorzugt sind. Besonders bevorzugt ist die Ausführung der Reaktion in Dimethylformamid in Gegenwart einer Base wie z.B. Natriumhydrid, Kaliumhydrid, 4-Dialkylaminopyridin oder tert. Aminen, besonders aber von Natriumhydrid.

Die Reaktion 3 zu 4 wird bei Temperaturen von -20°C bis zum Siedepunkt des verwendeten Lösungsmittels durchgeführt. Bevorzugt ist ein Temperaturbereich von -10 bis 60°C, besonders von 0 bis 30°C.

Die Verbindungen $R^3$-Z-C(O)-$R^a$ (5) lassen sich nach dem Fachmann bekannten Standardverfahren darstellen.

Eine bevorzugte Ausführungsform der Reaktion 3 zu 4 besteht in der Umsetzung von 3 mit Natriumhydrid in Dimethylformamid und anschließender Zugabe einer Lösung von $R^3$-Z-C(O)-Imidazol (5) in Dimethylformamid bei 0 bis 20°C, vorteilhafterweise unter Auschluß von Feuchtigkeit unter Schutzgas (Argon oder Stickstoff).

Die erhaltenen erfindungsgemäßen Verbindungen der Formel I können, soweit sie eine Carboxylgruppe enthalten, Salze mit anorganischen oder organischen Basen bilden. Bevorzugt sind daher auch solche Salze mit anorganischen Basen, besonders die physiologisch unbedenklichen Alkalimetall-Salze, vor allem Natrium- und Kaliumsalze.

Aus den Alkalimetallsalzen der Verbindungen der Formel I mit einer Carboxylgruppe können die für $R^1$ angegebenen Ester dargestellt werden. Hierzu wird Verbindung 4 in einem inerten organischen Lösungsmittel wie Tetrahydrofuran, Dimethylsulfoxid, bevorzugt Dimethylformamid bei -10 bis 60°C z. B. mit einem $C_1$-$C_4$-Alkylhalogenid, bevorzugt $C_1$-$C_4$-Alkyliodid, Benzylbromid, oder $C_1$-$C_4$-Alkanoyl-O-CH($R^4$)-Br oder $C_1$-$C_4$-Alkanoyl-O-CH($R^4$)-J zu den erfindungsgemäßen Verbindungen der Formel I mit einer Estergruppe als $R^1$ und X = $(CH_2)_m$ m=0 mit den zum Verfahren A genannten Details umgesetzt.

Verfahren B

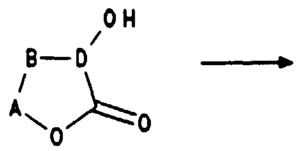

1

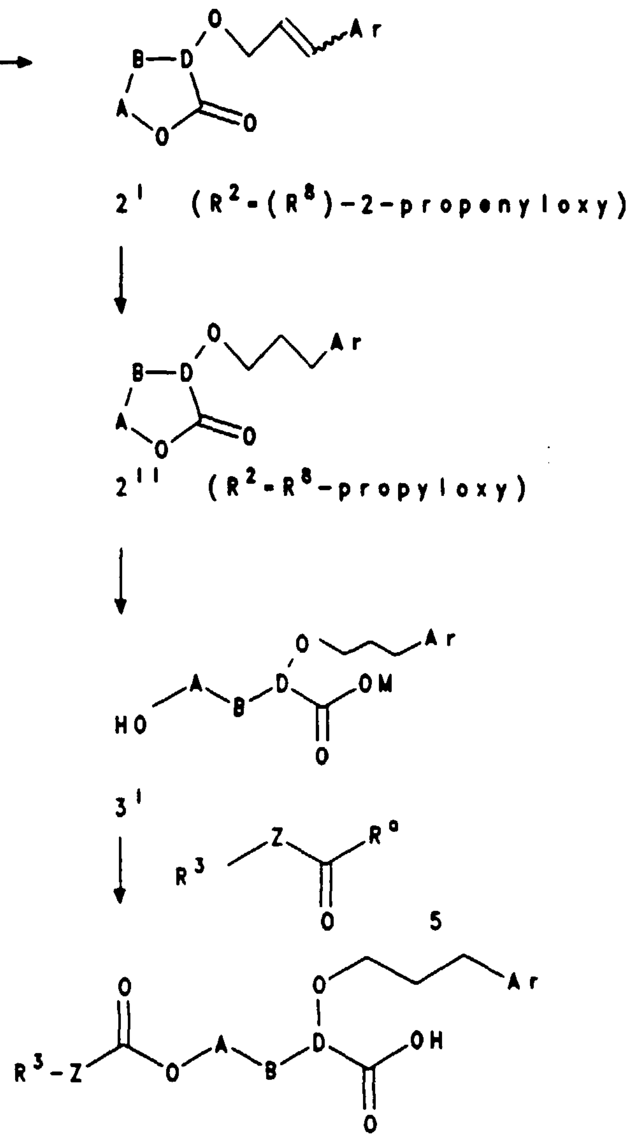

2' ($R^2$=($R^8$)-2-propenyloxy)

2'' ($R^2$=$R^8$-propyloxy)

3'

5

4'

Ar steht für einen aromatischen Rest $R^8$

(4' = Formel I, $R^2$ = $R^8$-propyloxy, übrige Bedeutungen vergl. Verfahren A)

Das Verfahren B ist dadurch charakterisiert, daß man die Verbindung 1 mit einer starken Base wie Kalium-tert.-butylat, Natriumhydrid oder Kaliumhydrid deprotoniert und zur Einführung von $R^2$ in der angegebenen Bedeutung mit entsprechenden Halogeniden, Trifluorsulfonsäureestern, Methylsulfonsäureestern oder p-Toluolsulfonsäureestern vorteilhafterweise in polaren aprotischen Lösungsmittel wie Dimethylformamid, Dimethylsulfoxid oder Tetrahydrofuran umsetzt, wobei Verbindung 2' entsteht. Bevorzugt wird die Verwendung von Natriumhydrid als Base und Dimethylformamid als Lösungsmittel.

Die Reaktion von 1 zu 2' wird bei Temperaturen von -20°C bis zum Siedepunkt des verwendeten Lösungsmittels durchgeführt. Bevorzugt ist ein Temperaturbereich von -10 bis 60°C, besonders von 0 bis 30°C.

Die bevorzugte Ausführungsform der Reaktion 1 zu 2' erfolgt in Dimethylformamid in Gegenwart von Natriumhydrid oder Kaliumhydrid bei Temperaturen von 0 bis 60°C. Die Reaktion wird dabei vorteilhafterweise unter Feuchtigkeitsausschluß unter einem Schutzgas (Stickstoff oder Argon) durchgeführt.

Die für die Umsetzung von 1 nach 2' notwendigen Ausgangsmaterialien, die dem Rest $R^2$ entsprechen, lassen sich nach dem für den Fachmann bekannten Standardverfahren darstellen. Hierbei handelt es sich um Strukturen des

Typs $R^{2'}$-E mit der für Verfahren B genannten Einschränkung (allerdings ohne das verknüpfende Sauerstoffatom). B hat z. B. die Bedeutung einer Abgangsgruppe wie Cl, Br, J, oder $OSO_2R$ (R = $CH_3$, Ph, Tolyl, $CF_3$).

Aus $2^I$ läßt sich durch Wasserstoff in Gegenwart von Hydrierkatalysatoren $2^{II}$ ($R^2$ = $R^8$-propyloxy) herstellen. Die Reaktionen werden in Ethylacetat oder Methanol bei Normaldruck durchgeführt. Die Reaktion wird mit einem Katalysator z.B. Rhodium auf Aluminiumoxid bei Temperaturen von 0°C bis zum Siedepunkt des verwendeten Lösungsmittels durchgeführt. Bevorzugt ist Ethylacetat als Lösungsmittel und ein Temperaturbereich von 20 bis 50°C, besonders von 20 bis 30°C.

Weiterer Schritt bei Verfahren B ist die Hydrolyse des Lactons $2^{II}$ zum Alkalisalz $3^I$ mit Alkalihydroxiden wie Lithiumhydroxid, Natriumhydroxid oder Kaliumhydroxid. Die Reaktion erfolgt vorteilhafterweise in protischen oder aprotischen Lösungsmitteln wie niederen Alkoholen, Tetrahydrofuran oder Dioxan, bevorzugt ist die Verwendung von Dioxan.

Die Reaktion von $2^{II}$ zu $3^I$ wird bei Temperaturen von -20°C bis zum Siedepunkt des verwendeten Lösungsmittels durchgeführt. Bevorzugt ist ein Temperaturbereich von -10 bis 60°C, besonders von 0 bis 30°C.

Weiterer Schritt ist die Reaktion von $3^I$ zu $4^I$, bei der der Rest $R^3$-Z-C(O)- an $3^I$ angebunden wird. Dazu wird $3^I$ in einem aprotischen organischen Lösungsmittel wie z.B. Tetrahydrofuran, Dimethylformamid, Dichlormethan, Pyridin oder Dimethylsulfoxid mit einer Verbindung $R^3$-Z-C(O)-$R^a$ (5) umgesetzt, wobei $R^a$ z.B. Cl, Br, OC(O)-$C_1$-$C_4$-Alkyl, Imidazolyl, Triazolyl oder Tetrazolyl sein kann, wobei Imidazolyl und Triazolyl besonders bevorzugt sind. Besonders bevorzugt ist die Ausführung der Reaktion in Dimethylformamid in Gegenwart einer Base wie z.B. Natriumhydrid, Kaliumhydrid, 4-Dialkylaminopyridin oder tert. Aminen, besonders aber von Natriumhydrid.

Die Reaktion $3^I$ zu $4^I$ wird bei Temperaturen von -20°C bis zum Siedepunkt des verwendeten Lösungsmittels durchgeführt. Bevorzugt ist ein Temperaturbereich von -10 bis 60°C, besonders von 0 bis 30°C.

Die Verbindungen $R^3$-Z-C(O)-$R^a$ (5) lassen sich nach dem Fachmann bekannten Standardverfahren darstellen.

Verfahren C (Definition für $R^a$ siehe Verfahren A)

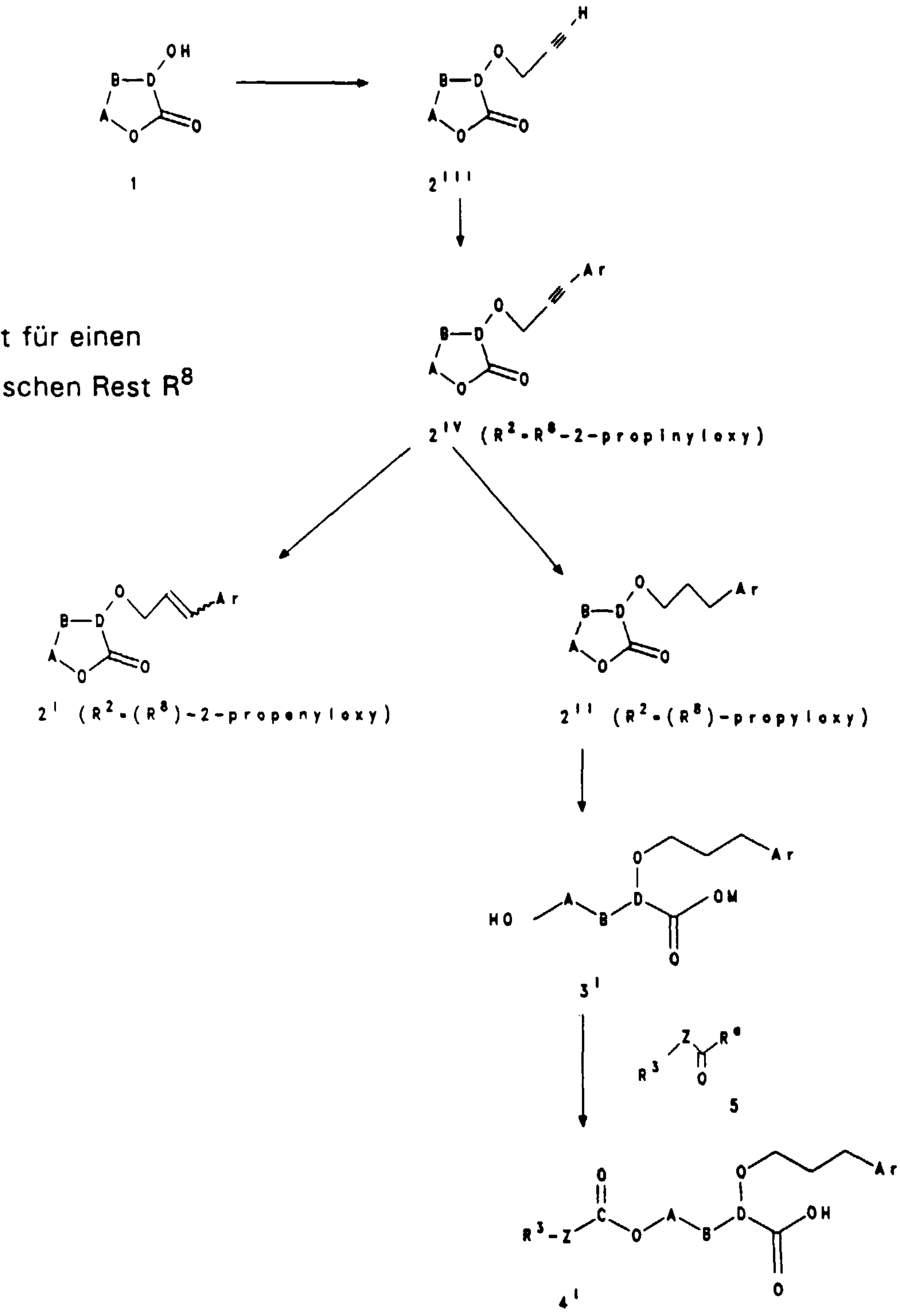

1

$2^{III}$

Ar steht für einen aromatischen Rest $R^8$

$2^{IV}$ ($R^2$=$R^8$-2-propinyloxy)

$2^{I}$ ($R^2$=($R^8$)-2-propenyloxy)

$2^{II}$ ($R^2$=($R^8$)-propyloxy)

$3^{I}$

5

$4^{I}$

Ein alternatives Verfahren zu A für eine Reihe erfindungsgemäßer Verbindungen der Formel I stellt das Verfahren C dar. Für $R^2$ = O-$C_3$-Alkinyl($R^8$) kann die Zwischenstufe $2^{III}$, bei der $R^2$ = 2-Propinyloxy entspricht, dienen. Hierbei wird $2^{III}$ ($R^2$ = 2-Propinyloxy) in einem inerten organischen Lösungsmittel wie z.B. Toluol, Benzol oder n-Heptan unter Katalyse eines Palladium-Komplexes und Kupfer(I)halogenid, besonders Kupfer(I)iodid, mit einem Arylhalogenid, besonders Arylbromid oder Aryliodid, zu $2^{IV}$ ($R^2$ = $R^8$-2-propinyloxy) umgesetzt. Hierzu muß eine Base wie z. B. primäre, sekundäre oder tertiäre Amine, besonders Triethylamin, zugegeben werden. Optional kann die Base auch gleichzeitig als Lösungsmittel dienen und auf den Zusatz eines weiteren organischen Lösungsmittels verzichtet werden.

Die Reaktion $2^{III}$ ($R^2$ = 2-Propinyloxy) zu $2^{IV}$ ($R^2$ = $R^8$-2-propinyloxy) wird bei Temperaturen von -20°C bis zum Siedepunkt des verwendeten Lösungsmittels durchgeführt. Bevorzugt ist ein Temperaturbereich von 20 bis 90°C, besonders von 60 bis 80°C.

Als Palladium-Komplex kann zum Beispiel der in situ aus Palladiumdichlorid und Triphenylphosphin darstellbare Komplex Ditriphenylphosphinpalladiumdichlorid oder der in gleicher Weise aus Palladium(II)acetat zugängliche Komplex Ditriphenylphosphinpalladiumdiacetat dienen, bevorzugt ist Ditriphenylphosphinpalladiumdichlorid.

Aus $2^{IV}$ ($R^2$ = $R^8$-2-propinyloxy) lassen sich durch Hydrierkatalysatoren gezielt $2^{I}$ ($R^2$ = $R^8$-2-propenyloxy) oder $2^{II}$ ($R^2$ = $R^8$-2-propyloxy) darstellen. Die Reaktionen durchgeführt.

Die Reaktion $2^{IV}$ ($R^2$ = $R^8$-2-propinyloxy) zu $2^{I}$ ($R^2$ = $R^8$-2-propenyloxy) wird mit einem Katalysator Palladium auf Bariumsulfat bei Temperaturen von 0°C bis zum Siedepunkt des verwendeten Lösungsmittels durchgeführt. Bevorzugt ist Pyridin als Lösungsmittel und ein Temperaturbereich von 20 bis 50°C, besonders von 20 bis 30°C.

Die Reaktion $2^{IV}$ ($R^2$ = $(R^8)$-2-propinyloxy) zu $2^{II}$ ($R^2$ = $(R^8)$propyloxy) wird mit Palladium auf Kohle als Katalysator in Ethanol bei Temperaturen von -20°C bis zum Siedepunkt des verwendeten Lösungsmittels durchgeführt. Bevorzugt ist ein Temperaturbereich von 20 bis 50°C, besonders von 20 bis 30°C.

Die weiteren Reaktionen von $2^{I}$ zu $4^{I}$ bzw. $2^{II}$ zu $4^{I}$, d.h. zu den Verbindungen der allgemeinen Formel I sind unter Verfahren B ausführlich beschrieben.

Die Verbindungen $2^{II}$ und $2^{IV}$ können auch ohne Hydrierungsschritte zu erfindungsgemäßen Verbindungen der Formel I ($R^2$ = O-$C_3$-Alkenyl($R^8$) bzw. $R^2$ = O-$C_3$-Alkinyl($R^8$)) analog Verfahren A (Schritte 2 und 3) umgesetzt werden.

Die Herstellung der Verbindung 5, die z.B. bei der Synthese der Verbindungen der Beispiele 1 bis 3 eingesetzt wird, erfolgt zweckmäßigerweise nach Verfahren I, II, III wie nachfolgend beschrieben.

Verfahren I

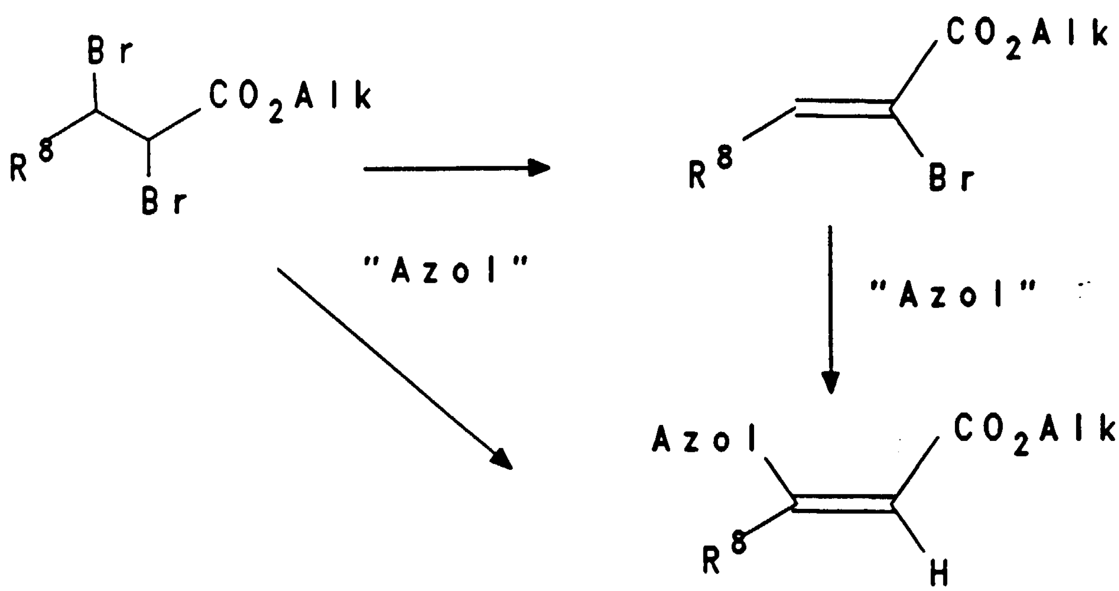


Verfahren II

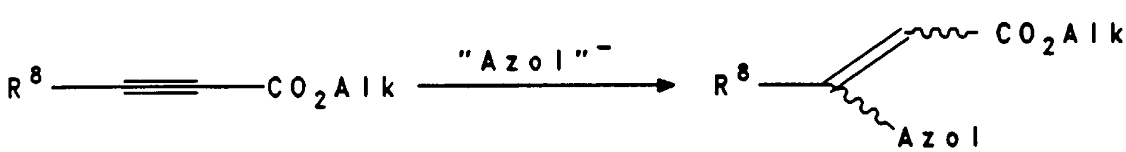

Verfahren III

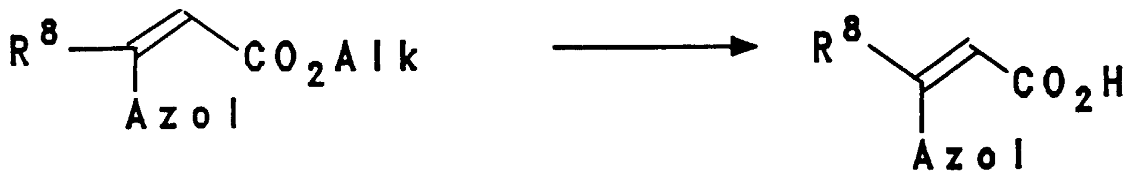

Alk steht für $C_1$-$C_4$-Alkyl
Azol steht für $R^{10}$ in der Bedeutung Imidazolyl, Indolyl, Piperazinyl, Tetrazolyl, Triazolyl oder deren thieno-, pyridino-, pyrimidino-, pyrazino-, pyridazino- oder benzoannelierte Derivate.

Verfahren I

Herstellung β-Azol-substituierter Zimtsäuremethylester

Ein Gemisch von 50 g 2,3-Dibrom-3-phenylpropansäuremethylester, 100 ml Triethylamin und 500 ml Toluol wird 1 h zum Sieden erhitzt, anschließend auf Raumtemperatur gekühlt und filtriert. Das Filtrat wird im Vakuum eingedampft und die so erhaltene α-Bromzimtsäure ohne Reinigung weiterverwendet. Zu einer Suspension von 4,7 g NaH (80 %ig in Mineralöl) in 100 ml wasserfreiem DMF werden 0,2 mol des Azol-Derivats, gelöst in 150 ml wasserfreiem DMF, unter Rühren zugetropft. Die Temperatur des Gemisches wird dabei durch Kühlung mit Eis unter 35°C gehalten. Nach beendeter Zugabe wird 1 h bei Raumtempertur gerührt. Die zuvor hergestellte α-Bromzimtsäure wird in 200 ml wasserfreiem DMF gelöst und unter Kühlung mit Eis die Lösung des Azol-Natriumsalzes unter Rühren zugetropft. Nach zweistündigem Rühren bei Raumtemperatur werden 10,8 ml Eisessig zugegeben, das Gemisch wird in 1,5 l Eiswasser eingerührt, mehrfach mit Ethylacetat extrahiert und die organischen Phasen werden mit Wasser gewaschen. Die organischen Phasen werden getrocknet, im Vakuum eingedampft und der Rückstand wird durch Säulenchromatographie an Kieselgel (Laufmittel: n-Heptan/Ethylacetat) oder Umkristallisieren gereinigt.

Verfahren II

Herstellung β-Azol-substituierter Zimtsäureethylester

Ein Gemisch aus 20 g Phenylpropiolsäureethylester, 0,11 mol Azol-Derivat und 15 ml wasserfreiem DMF wird bei Raumtemperatur unter Argon-Begasung gerührt. Ein Spatel NaH (80 %ig in Mineralöl) wird zugegeben. Wenn die Wasserstoffentwicklung beendet ist, wird auf 100-150°C (Badtemperatur) erwärmt und die Reaktion mit DC (Laufmittel n-Heptan/Ethylacetat) verfolgt. Nach beendeter Reaktion wird auf Raumtemperatur abgekühlt, im Vakuum eingeengt und der Rückstand aus n-Heptan umkristallisiert oder mit wenig n-Heptan/Ethylacetat verdünnt und durch Säulenchromatographie an Kieselgel (Laufmittel: n-Heptan/Ethylacetat) gereinigt.

Verfahren III

Herstellung β-Azol-substituierter Zimtsäuren aus β-Azol-substituierten Zimtsäureestern

In einer Lösung von 0,77 g NaOH in 50 ml Wasser und 10 ml Methanol werden 6,4 mmol β-Azol substituierter Zimtsäuremethyl- oder ethylester suspendiert und das Gemisch bei Raumtemperatur gerührt, bis im DC (Laufmittel n-Heptan/Ethylacetat) vollständiger Umsatz erkennbar ist und eine klare Lösung entstanden ist. Diese wird im Vakuum eingeengt, mit ca. 50 ml Wasser verdünnt und unter Eiskühlung mit 2 N HCl auf pH 2-3 gestellt. Falls ein Feststoff ausfällt, wird er abgesaugt und im Vakuum getrocknet. Andernfalls wird mit $CH_2Cl_2$ mehrfach extrahiert, die organischen Phasen werden getrocknet, im Vakuum eingedampft und der Rückstand wird durch Umkristallisieren oder Chromatographie an Kieselgel (Laufmittel: n-Heptan/Ethylacetat/Eisessig) gereinigt.
Ein weiterer Gegenstand der Erfindung sind Arzneimittel, die ein oder mehrere erfindungsgemäße Verbindungen der Formel I und/oder ihre pharmakologisch verträglichen Salze enthalten.
Die Arzneimittel werden nach an sich bekannten, dem Fachmann geläufigen Verfahren hergestellt. Als Arzneimittel werden die erfindungsgemäßen pharmakologisch wirksamen Verbindungen ( = Wirkstoff) entweder als solche, oder vorzugsweise in Kombination mit geegneten pharmazeutischen Hilfsstoffen in Form von Tabletten, Dragees, Kapseln, Suppositorien, Emulsionen, Suspensionen, Granulaten, Pulvern, Lösungen oder Präparate mit protalierter Wirkstoff-Freigabe, eingesetzt, wobei der Wirkstoffgehalt vorteilhafterweise 0,1 bis 95 % beträgt.

Welche Hilfstoffe für die gewünschte Arzneimittelformulierung geeignet sind, ist dem Fachmann aufgrund seines Fachwissens geläufig. Neben Lösungsmitteln, Gelbildnern, Suppositoriengrundlagen, Tabletten-Hilfsstoffen und anderen Wirkstoffträgern können beispielsweise Antioxidatien, Dispergiermittel, Emulgatoren, Entschäumer, Geschmackskorrigentien, Konservierungsmittel, Lösungsvermittler oder Farbstoffe verwendet werden.

Die Wirkstoffe können topisch, oral, parenteral oder intravenös appliziert werden, wobei die bevorzugte Applikationsart von der zu behandelnden Krankheit abhängig ist. Die orale Verabreichung ist bevorzugt.

Für eine orale Anwendungsform werden die aktiven Verbindungen mit den dafür geeigneten Zusatzstoffen wie Trägerstoffen, Stabilisatoren oder inerten Verdünndungsmittel vermischt und durch üblichen Methoden in geeigneten Dareichungsformen gebracht, wie Tabletten, Dragees, Steckkapseln, wässrige, alkoholische oder ölige Suspensionen oder wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen pflanzliche oder tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus verschiedenen Lösungsmitteln.

Als pharmazeutische Präparate für topische und lokale Verwendung eignen sich Augentropfen, welche die aktive Verbindung in wässriger oder öliger Lösung enthalten. Für die Anwendung an der Nase sind Aerosole und Sprays, sowie grobe Puder, die durch schnelles Inhalieren durch die Nasenlöcher verabreicht werden, und vor allem Nasentropfen, welche die aktive Verbindungen in wässriger oder öliger Lösung enthalten, geeignet.

Die Dosierung des zu verabreichenden Wirkstoffs der Formel I und die Häufigkeit der Verabreichung hängen von der Wirkstärke und Wirkdauer der verwendeten erfindungsgemäßen Verbindung ab; außerdem auch von der Art und Stärke der zu behandelnden Krankheit sowie von Geschlecht, Alter, Gewicht und individuellen Ansprechbarkeit des zu behandelnden Säugers. Im Durchschnitt liegt die empfohlene tägliche Dosis einer erfindungsgemäßen Verbindung bei einem etwa 75 Kg schweren Säuger - in erster Linier einem Menschen - im Bereich von etwa 10 bis 500 mg, vorzugweise von etwa 25 bis 250 mg, wobei die Verabreichung nach Bedarf in mehreren Dosen pro Tag erfolgen kann.

Die nachfolgenden Beispiele sollen die vorliegende Erfindung illustrieren, ohne sie jedoch in ihren Umfang einzuschränken.

Beispiel I (Verfahren B)

(±)-4-(3Z-(Benzimidazol-1-yl)-3-phenyl-propenoyloxy)-2-(4-chlorphenylpropyloxy)-3,3-dimethylbutansäure (I)

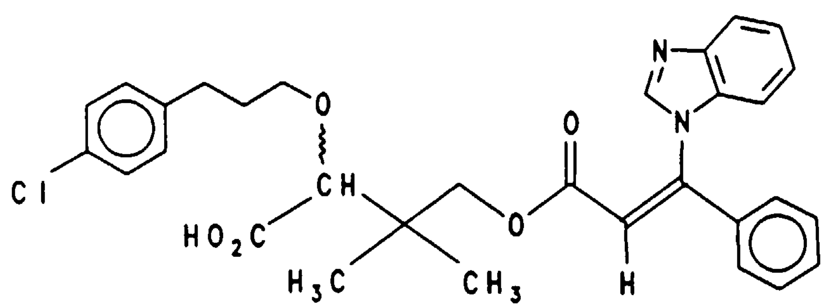

I

Stufe 1

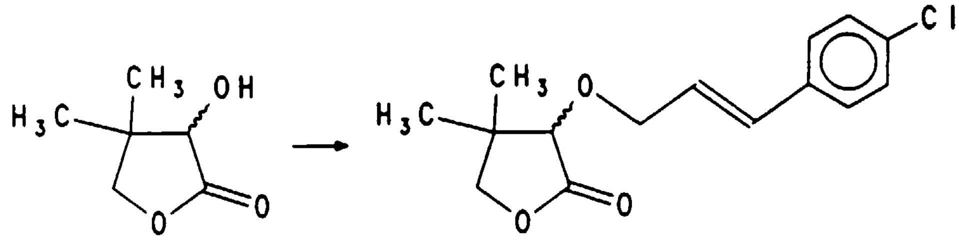

a b

Zu einer Lösung von 13,0 g DL-Pantholacton (a) in 300 ml wasserfreiem DMF werden bei 0°C unter Argon und unter Rühren portionsweise 3,3 g NaH 80-prozentig in Mineralöl innerhalb von einer Stunde zugegeben. Wenn die Gasentwicklung beendet ist, wird eine Lösung von 4-Chlorcinnamoylbromid in 100 ml wasserfreiem DMF tropfenweise innerhalb von 30 Minuten zugegeben. Der Reaktionsverlauf wird mittels DC (Fließmittel: Heptan/Ethylacetat) verfolgt. Nach beendeter Reaktion wird das Gemisch im Vakuum eingedampft, auf eiskalte konzentrierte $NH_4Cl$-Lösung gegossen, mehrfach mit $CH_2Cl_2$ extrahiert und die organischen Phasen getrocknet und eingedampft. Der Rückstand wird durch Chromatographie an Kieselgel (Fließmittel: Heptan-Ethylacetat-Gemisch) gereinigt und in Stufe 2 eingesetzt.

Stufe 2

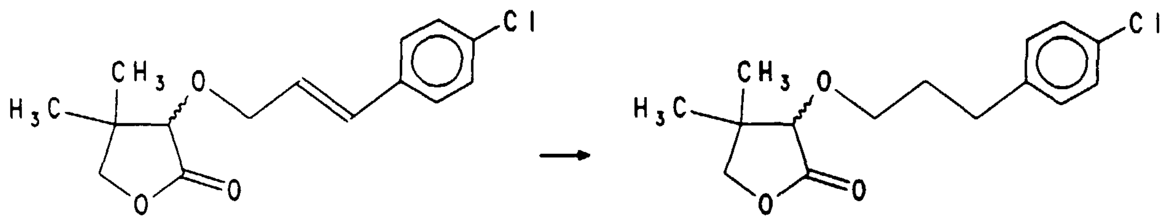

b c

Zu einer Lösung von 21,6 g Verbindung b in 1200 ml Ethylacetat werden 7,1 g Hydrierkatalysator Rhodium auf Aluminiumoxid 5-prozentig gegeben und in einer Hydrierapparatur bis zur theoretischen $H_2$-Aufnahme hydriert. Anschließend wird der Katalysator abfiltriert und das Filtrat eingedampft.

Stufe 3

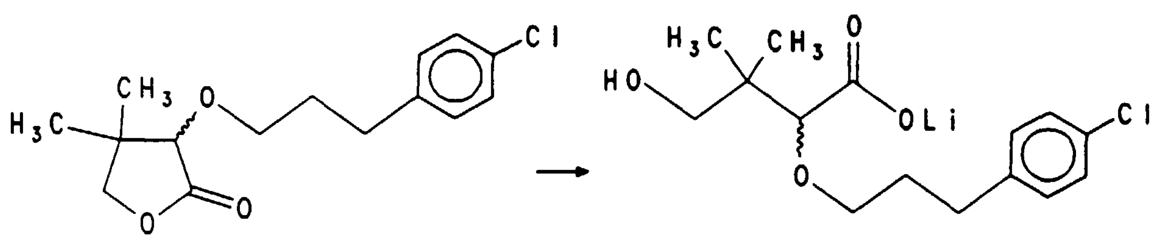

d

c

Zu einem Gemisch aus 2,82 g Verbindung c und 11 ml einer 1-molaren LiOH-Lösung in Wasser wird Dioxan gegeben, bis das Gemisch weitgehend homogen ist. Der Reaktionsverlauf wird mittels DC (Fließmittel: Heptan-Ethylacetat-Gemisch) verfolgt. Nach beendeter Reaktion wird das Gemisch im Vakuum eingedampft, der Rückstand in wasserfreiem DMF aufgenommen und erneut eingedampft.

## Stufe 4

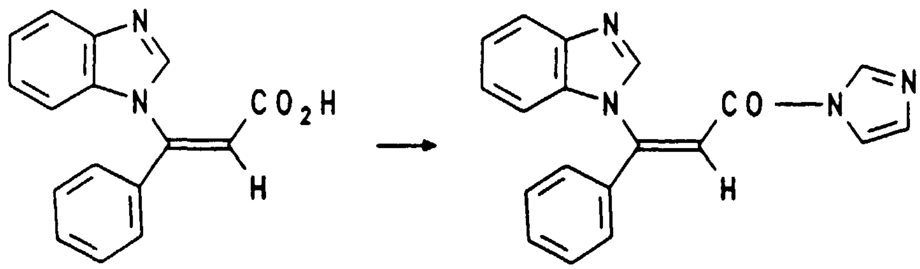

e f

Zu einer Lösung von 1,72 Carbonsäure e (Herstellung vergl. Verfahren I, II oder III), in 20 ml wasserfreiem DMF gibt man 1,18 g 1,1-Carbonyldiimidazol und rührt 5 Stunden unter Argon bei 50 bis 60°C. Das Produkt wird ohne Aufarbeitung direkt in Stufe 5 eingesetzt.

## Stufe 5

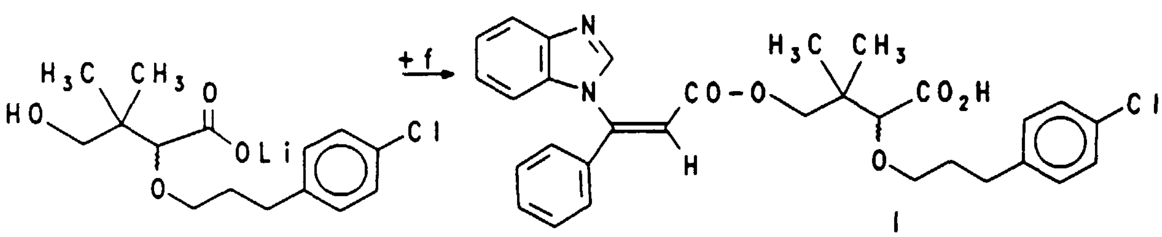


d I

Zu der nach Stufe 4 hergestellten Lösung von Verbindung f wird unter Argon zunächst 0,94 g Verbindung 10, hergestellt nach Stufe 3, zugegeben und anschließend 100 mg NaH 80-prozentig in Mineralöl zugegeben und das Gemisch etwa 1 Stunde bei Raumtemperatur gerührt. Die Reaktion wird mit DC (Fließmittel: Ethylacetat-Heptan-Eisessig-Gemisch) verfolgt. Zur Aufarbeitung wird das Gemisch auf eiskalte, gesättigte $NH_4Cl$-Lösung gegeben und mehrfach mit $CH_2Cl_2$ extrahiert. Die organischen Phasen werden getrocknet, im Vakuum eingedampft und der Rückstand wird durch Chromatographie an Kieselgel (Fließmittel: Ethylacetat-Heptan-Eisessig 40:20:1) gereinigt. Man erhält 1,36 g I als gelbliches Harz.
$^1$H-NMR (200 MHz, DMSO): $\delta$ = 0.8 (2 s); 1.6-1.9 (m); 2.5-2.7 (m); 3.1-3.9(m); 6.8 (s, überlagert mit d, J = 8 Hz); 7.1-7.6 (m); 7.75 (d, J = 8 Hz); 8.3 (s); 12.5 (bs) ppm.
MS (FAB): m/e = 547 (M + H$^+$)

Beispiel II (Verfahren B)

(±)-4-(3Z-(1H-Imidazo[4,5-b]pyridin-3-yl)-3-phenyl-propenoyloxy)-2-(4-chlorphenyl-propyloxy)-3,3-dimethylbutansäure (II)

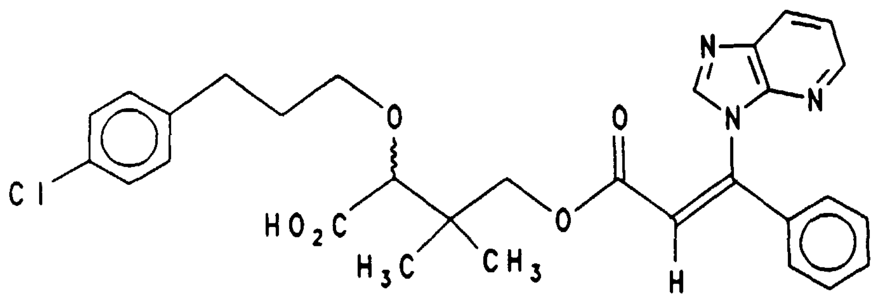

II

Stufe 1

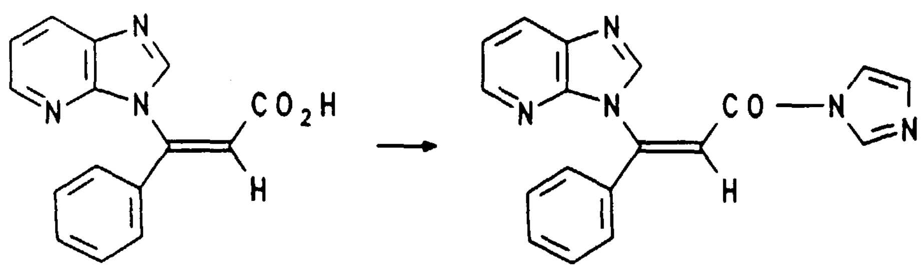

g h

Verbindung h wird analog Beispiel 1, Stufe 4 aus 1,8 g Verbindung g (Herstellung vergl. Verfahren I, II oder III) hergestellt und ohne Aufarbeitung direkt in Stufe 2 eingesetzt.

Stufe 2

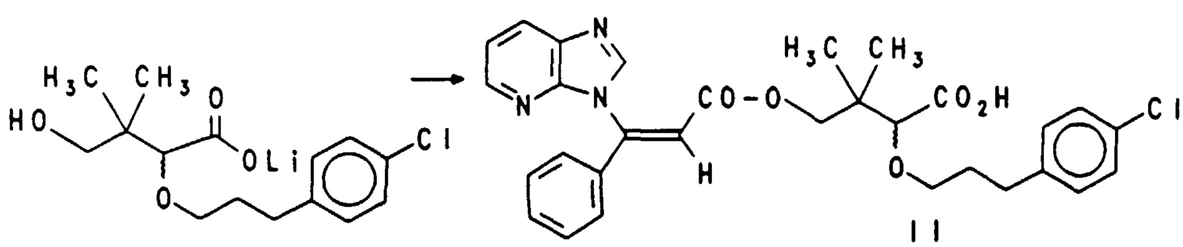

d II

Zu einer Lösung von 0,95 g Verbindung d (vergl. Beispiel 1, Stufe 1 bis 3) in 20 ml wasserfreiem DMF unter Argon werden 100 mg NaH 80-prozentig in Mineralöl gegeben und das Gemisch wird 2 Stunden bei Raumtemperatur gerührt. Danach wird die in Stufe 1 hergestellten Lösung von Verbindung h zugetropft und das Ganze 25 Stunden bei Raumtemperatur gerührt. Die Reaktion wird mit DC (Fließmittel: Ethylacetat-Heptan-Eisessig 30:30:1) verfolgt. Zur Aufarbeitung wird das Gemisch auf eiskalte, gesättigte $NH_4Cl$-Lösung gegeben und mehrfach mit $CH_2Cl_2$ extrahiert. Die organischen Phasen werden getrocknet, eingedampft und der Rückstand wird durch Chromatographie an Kieselgel

(Fließmittel: Ethylacetat-Heptan-Eisessig 10:50:1) gereinigt. Man erhält Verbindung II als gelbliches Harz.
$^1$H-NMR (200 MHz, DMSO): δ = 0.8 (s); 1.7 (m); 2.5 (m); 3.1-3.9 (m); 5.8 (s); 6.9 (s); 7.1-7.6 (m); 8.1 (m); 8.5 (s) ppm.
MS (FAB): m/e = 548 (M + $H^+$)

Beispiel III

(±)-4-(3Z-(1H-Imidazo[4,5-b]pyridin-3-yl)-3-phenyl-propenoyloxy)-2-(4-chlorphenyl-propyloxy)-butansäure (III)

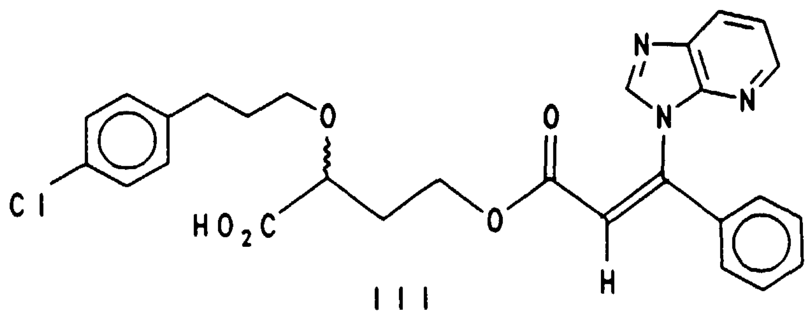

III

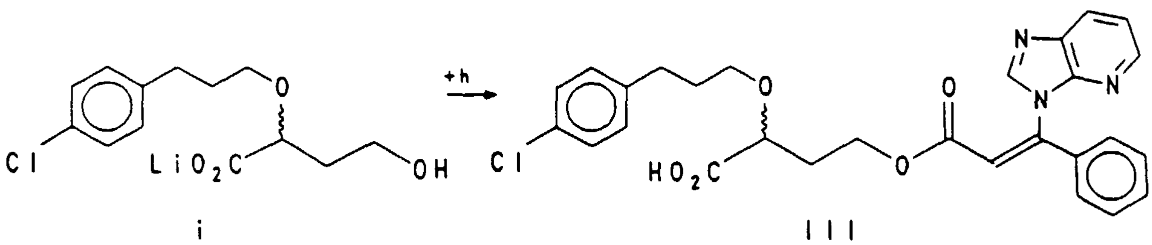


i

III

Verbindung i wird durch eine analoge Reaktionsfolge wie Beispiel 1, Stufe 1 bis 3 hergestellt, jedoch wird (±)-α-Hydroxybutyrolacton anstelle von DL-Pantholacton als Ausgangsmaterial eingesetzt.
Zu einer Lösung von 0,95 g Verbindung i in 20 ml wasserfreiem DMF unter Argon werden 100 mg NaH 80-prozentig in Mineralöl gegeben und das Gemisch wird 2 Stunden bei Raumtemperatur gerührt. Danach wird die wie in Beispiel 2, Stufe 1 aus 1,8 g Verbindung g hergestellte Lösung von Verbindung h zugetropft und 25 Stunden bei Raumtemperatur gerührt. Die Reaktion wird mit DC (Fließmittel: Ethylacetat-Heptan-Eisessig 30:30:1) verfolgt. Zur Aufarbeitung wird das Gemisch auf eiskalte, gesättigte $NH_4Cl$-Lösung zugegeben und mehrfach mit $CH_2Cl_2$ extrahiert. Die organischen Phasen werden getrocknet, eingedampft und der Rückstand wird durch Chromatographie an Kieselgel (Fließmittel: Ethylacetat-Heptan-Eisessig 30:30:1) gereinigt. Man erhält Verbindung III als gelblichen Feststoff, Fp. 95°C.
$^1$H-NMR (200 MHz, DMSO): δ = 1.7 (m); 2.6 (m); 3.1-3.7 (m); 4.0 (t); 6.85 (s); 7.1-7.6 (m); 8.1-8.3 (m); 8.5 (s); 12.5 (bs) ppm.
MS (FAB): m/e = 520 (M + $H^+$)

## Patentansprüche

**1.** Propan-Derivate der Formel I

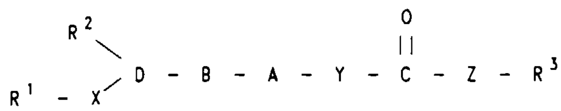 I

worin die Reste die folgenden Bedeutungen haben:

D-B-A: Propyl, das gegebenenfalls mit 1 bis 3 Gruppen ausgewählt aus $C_1$-$C_3$-Alkyl, ω-Hydroxy-($C_1$-$C_4$)-Alkyl, OH, O-$C_1$-$C_4$-Alkyl, Phenyl und Phenyl-$C_1$-$C_3$-Alkyl substituiert ist,

$R^1$: CN, COOH, eine mit einer Schutzgruppe geschützte COOH-Gruppe, $C_1$-$C_4$-Alkanoyl, $SO_3$-$C_1$-$C_4$-Alkyl, $SO_3H$, $SO_2NR^5R^6$, $PO(OH)_2$, PO(OH)(O-$C_1$-$C_4$-Alkyl), PO(O-$C_1$-$C_4$-Alkyl$)_2$, Tetrazol-5-yl,

$R^2$: $C_1$-$C_{10}$-Alkyl $(R^8)_n$, O-$C_1$-$C_{10}$-Alkyl $(R^8)_n$,
$C_2$-$C_{10}$-Alkenyl $(R^8)_n$, O-$C_3$-$C_{10}$-Alkenyl $(R^8)_n$,
$C_2$-$C_{10}$-Alkinyl $(R^8)_n$, O-$C_3$-$C_{10}$-Alkinyl $(R^8)_n$,
S-$C_1$-$C_{10}$-Alkyl $(R^8)_n$, S-$C_3$-$C_{10}$-Alkenyl $(R^8)_n$,
S-$C_3$-$C_{10}$-Alkinyl $(R^8)_n$,
NH-$C_1$-$C_{10}$-Alkyl $(R^8)_n$, NH-$C_3$-$C_{10}$-Alkenyl $(R^8)_n$ oder
NH-$C_3$-$C_{10}$-Alkinyl $(R^8)_n$, wobei $R^8$ gegebenenfalls jeweils mit $R^9$ substituiert ist;

$R^3$, $R^8$ und $R^{10}$: Alkyl mit 1 bis 10 C-Atomen, Cycloalkyl mit 3-8 Ring-C-Atomen, Phenyl, Naphthyl, Phenanthryl, Pyridyl, Thienyl, Furyl, Pyrimidyl, Indolyl, Imidazolyl, Coumarinyl, Phthalimidyl, Chinolyl, Piperazinyl, Tetrazolyl, Triazolyl, Oxazolyl oder deren thieno-, pyridino-, pyrimidino-, pyrazino-, pyridazino oder benzoanellierte Derivate, wobei der Aromat bzw. Heteroaromat ein- oder mehrfach mit F, Cl, Br, J, OH, $CF_3$, $-NO_2$, CN, $C_1$-$C_4$-Alkoxy, $C_1$-$C_4$-Alkyl, $NR^5R^6$, Phenyl, Benzyl, Thienyl, Furyl, Imidazolyl, Pyridyl, 0-Phenyl oder 0-Benzyl gleich oder verschieden substituiert sein kann, und $R^3$, $R^8$ und $R^{10}$ gleich oder verschieden sind,

$R^4$: $C_1$-$C_4$-Alkyl, Phenyl oder Benzyl;

$R^5$ und $R^6$: H, $C_1$-$C_4$-Alkyl, $C_1$-$C_4$-Alkanoyl, Phenyl, das gegebenenfalls substituiert ist mit F, Cl, Br, J, OH, O-$C_1$-$C_4$-Alkyl, $CF_3$, $-NO_2$ oder CN, wobei $R^5$ und $R^6$ gleich oder verschieden sind, oder $R^5$ und $R^6$ bilden zusammen mit dem Stickstoffatom einen 4 bis 10gliedrigen, gesättigten heterocyclischen Ring, bei dem gegebenenfalls eine $CH_2$-Gruppe durch O, S oder $NR^7$ ersetzt sein kann,

$R^7$: H, $C_1$-$C_4$-Alkyl, Phenyl oder Benzyl,

$R^9$: Phenyl, Naphthyl, Phenanthryl, Pyridyl, Thienyl, Furyl, Pyrimidyl, Indolyl, Imidazolyl, Coumarinyl, Phthalimidyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, J, OH, $CF_3$, $-NO_2$, CN, $C_1$-$C_4$-Alkoxy, $C_1$-$C_4$-Alkyl, $C_2$-$C_4$-Alkenyl, $NR^5R^6$, Phenyl, Benzyl, Thienyl, Furyl, Imidazolyl, Pyridyl, O-Phenyl oder 0-Benzyl gleich oder verschieden substituiert sein kann,

X: $(CH_2)_m$, -CH=CH-, -C≡C-, $-CH_2-O-CH_2-$, $-CH_2-S-CH_2-$ oder

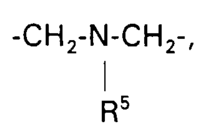

Y: $(CH_2)_m$, O, S, $NR^5$,

Z: $(CH_2)_m$, S, O, S-$C_1$-$C_{10}$-Alkyl, O-$C_1$-$C_{10}$-Alkyl, CH=CH, CH=CF, CH=CCl, CH=CBr, $CH_2$-CO, $CH_2$-CHF, $CH_2$-CHCl, $CH_2$-CHBr, $CH_2$-CHJ, $C_3$-$C_{10}$-Cycloalkylen, $C_3$-$C_{10}$-Cycloalkenylen, wobei 1 bis 3 Ring-C-Atome durch Schwefel-, Sauerstoff- oder Stickstoffatome ersetzt sein können, $COOR^4$, C≡C, CH =C($C_1$-$C_4$-Alkyl), CH = C(CN), CH =C($NR^5R^6$), CH =C($C_1$-$C_4$-Alkanoyl), CH=C($R^{10}$), $NR^5$ und wenn Y Sauerstoff ist, kann

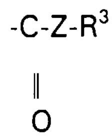

gemeinsam einen Aminosäurerest, ausgewählt aus der Gruppe bestehend aus Ala, Arg, Asn, Asp, Cys, Gln, Glu, Gly, His, Ile, Leu, Lys, Phe, Pro, Ser, Thr, Trp, Tyr und deren durch übliche Schutzgruppen geschützte Derivate, darstellen,

n: null, 1 oder 2

m: null, 1, 2, 3 oder 4,

sowie physiologisch verträgliche Salze von Verbindungen der Formel I.

**2.** Verbindungen gemäß Anspruch 1, dadurch gekennzeichnet, daß in Formel I

$R^1$ CN, COOH, eine mit einer Schutzgruppe geschützte COOH-Gruppe oder $C_1$-$C_4$-Alkanoyl bedeutet und die übrigen Reste die in Anspruch 1 angegebenen Bedeutungen haben.

**3.** Verbindungen gemäß Anspruch 1, dadurch gekennzeichnet, daß in Formel I die Reste folgende Bedeutungen haben:

$R^1$: CN, COOH, eine mit einer Schutzgruppe geschützte COOH-Gruppe oder $C_1$-$C_4$-Alkanoyl oder Tetrazol-5-yl,

$R^2$: O-$C_1$-$C_{10}$-Alkyl$(R^8)_n$ (n=0,1,2), wobei der Alkylteil unverzweigt, verzweigt oder cyclisch ist, sowie eine der $CH_2$-Gruppen durch O ersetzt sein kann und $R^8$ mit $R^9$ substituiert sein kann und bei n = 2 die beiden Reste $R^8$ gleich oder verschieden sind,
O-$C_3$-$C_{10}$-Alkenyl$(R^8)_n$ (n=0,1,2), wobei der Alkenylteil unverzweigt, verzweigt oder cyclisch ist, eine der $CH_2$-Gruppen durch O, S, SO, $SO_2$ oder $NR^5$ ersetzt sein kann sowie ein- oder mehrfach ungesättigt ist, und $R^8$ mit $R^9$ substituiert sein kann und bei n = 2 die beiden Reste $R^8$ gleich oder verschieden sind,
O-$C_3$-$C_{10}$-Alkinyl$(R^8)_n$ (n=0,1,2), wobei der Alkinylteil unverzweigt, verzweigt oder cyclisch ist, ein- oder mehrfach ungesättigt ist sowie eine der $CH_2$-Gruppen durch O, S, SO, $SO_2$, oder $NR^5$ ersetzt sein kann und $R^8$ mit $R^9$ substituiert sein kann und bei n = 2 die beiden Reste $R^8$ gleich oder verschieden sind,

$R^3$ bis $R^{10}$ die in Anspruch 1 angegebenen Bedeutungen haben,

X: $(CH_2)_m$, (m=0,1,2,3,4) CH=CH, C≡C, $CH_2OCH_2$, $CH_2SCH_2$

Y: $(CH_2)_m$, (m=0,1,2,3,4) O, S, $NR^5$,

Z: $(CH_2)_m$, (m=0,1,2,3,4) S, O, S-$C_1$-$C_{10}$-Alkyl, (unverzweigt oder

verzweigt), CH=CH, CH=CF, CH=CCl, CH=CBr, $CH_2$-C(O), $CH_2$-CHF, $CH_2$-CHCl, $CH_2$-CHBr, $CH_2$-CHJ, $C_3$-$C_{10}$-Cycloalkylen, $C_3$-$C_{10}$-Cycloalkenylen, $COOR^4$, C≡C, CH=C($C_1$-$C_4$-Alkyl) (unverzweigt oder verzweigt), CH=C (CN), CH=C($R^{10}$), $NR^5$.

**4.** Verbindungen gemäß Anspruch 1, dadurch gekennzeichnet, daß in Formel I D-B-A unsubstituiertes oder an B mit 2 Methylgruppen substituiertes Propyl bedeutet.

**5.** Anwendung von Verbindungen nach Anspruch 1 zur Herstellung von Arzneimitteln zur Behandlung des Typ II

Diabetes und anderer Erkrankungen, die durch einen erhöhten Glucose-Ausstoß aus der Leber oder eine erhöhte Aktivität des Glucose-6-Phosphatase-Systems gekennzeichnet sind.

**6.** Arzneimittel enthaltend eine Verbindung nach Anspruch 1.

**Claims**

**1.** A propane derivative of the formula I

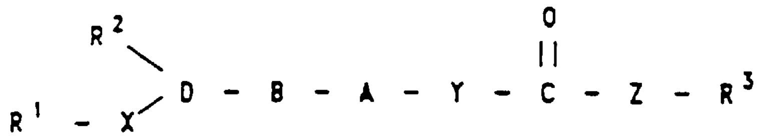

I

in which the radicals have the following meanings:

D-B-A: propanetriyl which is optionally substituted by 1 to 3 groups selected from $C_1$-$C_3$-alkyl, ω-hydroxy-($C_1$-$C_4$)-alkyl, OH, O-$C_1$-$C_4$-alkyl, phenyl and phenyl-$C_1$-$C_3$-alkyl,

$R^1$: CN, COOH, a COOH group protected with a protective group, $C_1$-$C_4$-alkanoyl, $SO_3$-$C_1$-$C_4$-alkyl, $SO_3H$, $SO_2NR^5R^6$, $PO(OH)_2$, PO(OH) (O-$C_1$-$C_4$-alkyl), PO(O-$C_1$-$C_4$-alkyl$)_2$, 5-tetrazolyl,

$R^2$: $C_1$-$C_{10}$-alkyl$(R^8)_n$, O-$C_1$-$C_{10}$-alkyl$(R^8)_n$,
$C_2$-$C_{10}$-alkenyl$(R^8)_n$, O-$C_3$-$C_{10}$-alkenyl$(R^8)_n$,
$C_2$-$C_{10}$-alkynyl$(R^8)_n$, O-$C_3$-$C_{10}$-alkynyl$(R^8)_n$,
S-$C_1$-$C_{10}$-alkyl $(R^8)_n$, S-$C_3$-$C_{10}$-alkenyl$(R^8)_n$,
S-$C_3$-$C_{10}$-alkynyl$(R^8)_n$, NH-$C_1$-$C_{10}$-alkyl$(R^8)_n$,
NH-$C_3$-$C_{10}$-alkenyl$(R^8)_n$ or NH-$C_3$-$C_{10}$-alkynyl$(R^8)_n$,
where $R^8$ is optionally in each case substituted by $R^9$;

$R^3$, $R^8$ and $R^{10}$: alkyl having 1 to 10 carbon atoms, cycloalkyl having 3-8 ring carbon atoms, phenyl, naphthyl, phenanthryl, pyridyl, thienyl, furyl, pyrimidyl, indolyl, imidazolyl, coumarinyl, phthalimidyl, quinolyl, piperazinyl, tetrazolyl, triazolyl, oxazolyl or their thieno-, pyridino-, pyrimidino-, pyrazino-, pyridazino- or benzo-fused derivatives, where the aromatic or heteroaromatic system can be substituted one or more times, identically or differently, by F, Cl, Br, I, OH, $CF_3$, $-NO_2$, CN, $C_1$-$C_4$-alkoxy, $C_1$-$C_4$-alkyl, $NR^5R^6$, phenyl, benzyl, thienyl, furyl, imidazolyl, pyridyl, O-phenyl or O-benzyl, and $R^3$, $R^8$ and $R^{10}$ are identical or different;

$R^4$: $C_1$-$C_4$-alkyl, phenyl or benzyl;

$R^5$ and $R^6$: H, $C_1$-$C_4$-alkyl, $C_1$-$C_4$-alkanoyl, phenyl which is optionally substituted by F, Cl, Br, I, OH, O-$C_1$-$C_4$-alkyl, $CF_3$, $-NO_2$ or CN, where $R^5$ and $R^6$ are identical or different, or $R^5$ and $R^6$ form, together with the nitrogen atom, a 4- to 10-membered, saturated heterocyclic ring in which one $CH_2$ group can optionally be replaced by O, S or $NR^7$,

$R^7$: H, $C_1$-$C_4$-alkyl, phenyl or benzyl;

$R^9$: phenyl, naphthyl, phenanthryl, pyridyl, thienyl, furyl, pyrimidyl, indolyl, imidazolyl, coumarinyl, phthalimidyl, quinolyl, piperazinyl, tetrazolyl, triazolyl, oxazolyl or their thieno- or benzo-fused derivatives, where the aromatic or heteroaromatic system can be substituted one or more times, identically or differently, by F, Cl, Br, I, OH, $CF_3$, $-NO_2$, CN, $C_1$-$C_4$-alkoxy, $C_1$-$C_4$-alkyl, $C_2$-$C_4$-alkenyl, $NR^5R^6$, phenyl, benzyl, thienyl, furyl, imidazolyl, pyridyl, O-phenyl or O-benzyl;

X: $(CH_2)_m$, -CH=CH-, -C≡C-, $-CH_2-O-CH_2-$, $-CH_2-S-CH_2-$ or

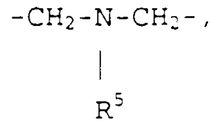

Y: $(CH_2)_m$, O, S, $NR^5$,

Z: $(CH_2)_m$, S, O, S-$C_1$-$C_{10}$-alkyl, O-$C_1$-$C_{10}$-alkyl, CH=CH, CH=CF, CH=CCl, CH=CBr, $CH_2$-CO, $CH_2$-CHF, $CH_2$-CHCl, $CH_2$-CHBr, $CH_2$-CHI, $C_3$-$C_{10}$-cycloalkylene, $C_3$-$C_{10}$-cycloalkenylene, where 1 to 3 ring carbon atoms can be replaced by sulfur, oxygen or nitrogen atoms, $COOR^4$, C≡C, CH=C($C_1$-$C_4$-alkyl), CH=C(CN), CH=C($NR^5R^6$), CH=C ($C_1$-$C_4$-alkanoyl), CH=C($R^{10}$), $NR^5$ and, when Y is oxygen, it is possible for

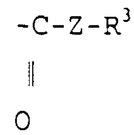

together to represent an amino-acid residue selected from the group consisting of Ala, Arg, Asn, Asp, Cys, Gln, Glu, Gly, His, Ile, Leu, Lys, Phe, Pro, Ser, Thr, Trp, Tyr and their derivatives protected by customary protective groups,

n: zero, 1 or 2

m: zero, 1, 2, 3 or 4,

and physiologically tolerated salts of compounds of the formula I.

**2.** A compound as claimed in claim 1, wherein

$R^1$ in formula I is CN, COOH, a COOH group protected with a protective group, or $C_1$-$C_4$-alkanoyl, and the other radicals have the meanings stated in claim 1.

**3.** A compound as claimed in claim 1, wherein the radi cals in formula I have the following meanings:

$R^1$ : CN, COOH, a COOH group protected by a protective group or $C_1$-$C_4$-alkanoyl or 5-tetrazolyl,
$R^2$: O-$C_1$-$C_{10}$-alkyl$(R^8)_n$ (n = 0, 1, 2), where the alkyl moiety is unbranched, branched or cyclic, and one of the $CH_2$ groups can be replaced by O, and $R^8$ can be substituted by $R^9$, and, when n = 2, the two $R^8$ radicals are identical or different,
O-$C_3$-$C_{10}$-alkenyl$(R^8)_n$ (n = 0, 1, 2), where the alkenyl moiety is unbranched, branched or cyclic, one of the $CH_2$ groups can be replaced by O, S, SO, $SO_2$ or $NR^5$, and is mono- or polyunsaturated, and $R^8$ can be substituted by $R^9$, and, when n = 2, the two $R^8$ radicals are identical or different,
O-$C_3$-$C_{10}$-alkynyl$(R^8)_n$ (n = 0, 1, 2), where the alkynyl moiety is unbranched, branched or cyclic, and is mono- or polyunsaturated, and one of the $CH_2$ groups can be replaced by O, S, SO, $SO_2$ or $NR^5$, and $R^8$ can be substituted by $R^9$, and, when n = 2, the two $R^8$ radicals are identical or different,
$R^3$ to $R^{10}$ have the meanings stated in claim 1,
X: $(CH_2)_m$, (m = 0, 1, 2, 3, 4) CH=CH, C≡C, $CH_2OCH_2$, $CH_2SCH_2$
Y: $(CH_2)_m$, (m = 0, 1, 2, 3, 4) O, S, $NR^5$,
Z: $(CH_2)_m$, (m = 0, 1, 2, 3, 4) S, O, S-$C_1$-$C_{10}$-alkyl, (unbranched or branched), CH=CH, CH=CF, CH=CCl, CH=CBr, $CH_2$-C(O), $CH_2$-CHF, $CH_2$-CHCl, $CH_2$-CHBr, $CH_2$-CHI, $C_3$-$C_{10}$-cycloalkylene, $C_3$-$C_{10}$-cycloalkenylene, $COOR^4$, C≡C, CH=C($C_1$-$C_4$-alkyl) (unbranched or branched), CH=C(CN), CH=C ($R^{10}$), $NR^5$.

**4.** A compound as claimed in claim 1, wherein
D-B-A in formula I is propyl which is unsubstituted or substituted on B by 2 methyl groups.

5. The use of compounds as claimed in claim 1 for the production of pharmaceuticals for the treatment of type II diabetes and other disorders characterized by increased output of glucose from the liver or an increased activity of the glucose-6-phosphatase system.

6. A pharmaceutical containing a compound as claimed in claim 1.

## Revendications

1. Dérivés de propane de formule I

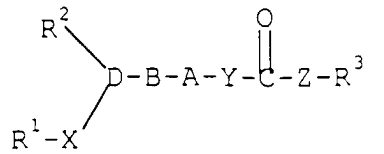

I

dans laquelle les radicaux ont les significations suivantes :

| | |
|---|---|
| D-B-A: | un radical propylène qui est éventuellement substitué par un à trois groupes choisis parmi des groupes alkyle en $C_1$-$C_3$, ω-hydroxyalkyle($C_1$-$C_4$), OH, O-alkyle($C_1$-$C_4$), phényle et phénylalkyle($C_1$-$C_3$), |
| $R^1$ : | CN, COOH, un groupe COOH protégé par un groupe protecteur, alcanoyle en $C_1$-$C_4$, $SO_3$-alkyle($C_1$-$C_4$), $SO_3H$, $SO_2NR^5R^6$, $PO(OH)_2$, PO(OH)(O-alkyle[$C_1$-$C_4$]), PO(O-alkyle[$C_1$-$C_4$])$_2$, tétrazol-5-yle, |
| $R^2$ : | alkyl($C_1$-$C_{10}$)-($R^8$)$_n$, O-alkyl($C_1$-$C_{10}$)-($R^8$)$_n$, alcényl($C_2$-$C_{10}$)-($R^8$)$_n$, O-alcényl($C_3$-$C_{10}$)-($R^8$)$_n$, alcynyl($C_2$-$C_{10}$)-($R^8$)$_n$, O-alcynyl($C_3$-$C_{10}$)-($R^8$)$_n$, S-alkyl($C_1$-$C_{10}$)-($R^8$)$_n$, S-alcényl($C_3$-$C_{10}$)-($R^8$)$_n$, S-alcynyl-($C_3$-$C_{10}$)-($R^8$)$_n$, NH-alkyl($C_1$-$C_{10}$)-($R^8$)$_n$, NH-alcényl($C_3$-$C_{10}$)-($R^8$)$_n$ ou NH-alcynyl($C_3$-$C_{10}$)-($R^8$)$_n$, $R^8$ étant dans chaque cas éventuellement substitué par $R^9$ ; |
| $R^3$, $R^8$ et $R^{10}$ : | un groupe alkyle ayant de 1 à 10 atomes de carbone, cycloalkyle ayant 3-8 atomes de carbone formant le cycle, phényle, naphtyle, phénanthryle, pyridyle, thiényle, furyle, pyrimidyle, indolyle, imidazolyle, coumarinyle, phtalimidyle, quinolyle, pipérazinyle, tétrazolyle, triazolyle, oxazolyle ou leurs dérivés condensés à un noyau thiéno, pyridino, pyrimidino, pyrazino, pyridazino ou benzo, le fragment aromatique ou hétéroaromatique pouvant être une ou plusieurs fois substitué par un ou des atomes de F, C1, Br, I ou groupes OH, $CF_3$, $-NO_2$, CN, alcoxy en $C_1$-$C_4$, alkyle en $C_1$-$C_4$, $NR^5R^6$, phényle, benzyle, thiényle, furyle, imidazolyle, pyridyle, O-phényle ou O-benzyle, identiques ou différents, et $R^3$, $R^8$ et $R^{10}$ étant identiques ou différents ; |
| $R^4$: | un groupe alkyle en $C_1$-$C_4$, phényle ou benzyle ; |
| $R^5$ et $R^6$ : | H ou un groupe alkyle en $C_1$-$C_4$, alcanoyle en $C_1$-$C_4$, phényle, qui est éventuellement substitué par F, Cl, Br, I, OH, un groupe O-alkyle($C_1$-$C_4$), $CF_3$, $-NO_2$ ou CN, $R^5$ et $R^6$ étant identiques ou différents, ou $R^5$ et $R^6$ forment ensemble, conjointement avec l'atome d'azote, un cycle saturé hétérocyclique à 4-10 chaînons, dans lequel un groupe CH2 peut éventuellement être remplacé par O, S ou $NR^7$, |
| $R^7$: | H ou un groupe alkyle en $C_1$-$C_4$, phényle ou benzyle ; |
| $R^9$ : | un radical phényle, naphtyle, phénanthryle, pyridyle, thiényle, furyle, pyrimidyle, indolyle, imidazolyle, coumarinyle, phtalimidyle, quinolyle, pipérazinyle, tétrazolyle, triazolyle, oxazolyle ou leurs dérivés condensés à un noyau thiéno ou benzo, le fragment aromatique ou hétéroaromatique pouvant être une ou plusieurs fois substitué par un ou des atomes de F, C1, Br, I ou groupes OH, $CF_3$, $-NO_2$, CN, alcoxy en $C_1$-$C_4$, alkyle en $C_1$-$C_4$, alcényle en $C_2$-$C_4$, $NR^5R^6$, phényle, benzyle, thiényle, furyle, imidazolyle, pyridyle, O-phényle ou O-benzyle, identiques ou différents ; |
| X : | $(CH_2)_m$, -CH=CH-, -C≡C-, $-CH_2-O-CH_2-$, $-CH_2-S-CH_2-$ ou |

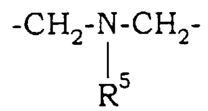

Y: $(CH_2)_m$, O, S, $NR^5$,

Z: $(CH_2)_m$, S, O, S-alkyle($C_1$-$C_{10}$), O-alkyle($C_1$-$C_{10}$), CH=CH, CH=CF, CH=CCl, CH=CBr, $CH_2$-CO, $CH_2$-CHF, $CH_2$-CHCl, $CH_2$-CHBr, $CH_2$-CHI, cycloalkylène en $C_3$-$C_{10}$, cycloalcénylène en $C_3$-$C_{10}$, 1 à 3 atomes de carbone formant le cycle pouvant être remplacés par un ou des atomes de soufre, oxygène ou azote, $COOR^4$, C≡C, CH=C(alkyle[$C_1$-$C_4$]), CH=C(CN), CH=C($NR^5R^6$), CH=C(alcanoyl[$C_1$-$C_4$]), CH=C($R^{10}$), $NR^5$, et, lorsque Y est un atome d'oxygène,

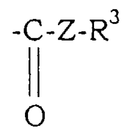

peut représenter dans son ensemble un reste d'aminoacide choisi dans l'ensemble constitué par Ala, Arg, Asn, Asp, Cys, Gln, Glu, Gly, His, Ile, Leu, Lys, Phe, Pro, Ser, Thr, Trp, Tyr et leurs dérivés protégés par des groupes protecteurs usuels,

n = zéro, 1 ou 2,

m = zéro, 1, 2, 3 ou 4,

et sels physiologiquement acceptables des composés de formule I.

**2.** Composés selon la revendication 1, caractérisés en ce que, dans la formule I, $R^1$ représente CN, COOH, un groupe COOH protégé par un groupe protecteur, ou un groupe alcanoyle en $C_1$-$C_4$, et les autres radicaux ont les significations données dans la revendication 1.

**3.** Composés selon la revendication 1, caractérisés en ce que, dans la formule I, les radicaux ont les significations suivantes :

$R^1$ : CN, COOH, un groupe COOH protégé par un groupe protecteur, ou un groupe alcanoyle en $C_1$-$C_4$ ou tétrazol-5-yle,

$R^2$ : un groupe O-alkyl($C_1$-$C_{10}$)-$(R^8)_n$ (n = 0, 1, 2), dans lequel le fragment alkyle est non ramifié, ramifié ou cyclique, et l'un des groupes $CH_2$ peut être remplacé par O, et $R^8$ peut être substitué par $R^9$, et, dans le cas où n = 2, les deux radicaux $R^8$ sont identiques ou différents, un groupe O-alcényl($C_3$-$C_{10}$)-$(R^8)_n$ (n = 0, 1, 2), le fragment alcényle étant non ramifié, ramifié ou cyclique, l'un des groupes $CH_2$ peut être remplacé par O, S, SO, $SO_2$ ou $NR^5$, ainsi qu'une ou plusieurs fois insaturé, et $R^8$ peut être substitué par $R^9$, et dans le cas où n = 2, les deux radicaux $R^8$ sont identiques ou différents,
un groupe O-alcynyl($C_3$-$C_{10}$)-$(R^8)_n$ (n = 0, 1, 2), dans lequel le fragment alcynyle est non ramifié, ramifié ou cyclique, est une ou plusieurs fois insaturé, et l'un des groupes $CH_2$ peut être remplacé par O, S, SO, $SO_2$ ou $NR^5$, et $R^8$ peut être substitué par $R^9$, et, dans le cas où n = 2, les deux radicaux $R^8$ sont identiques ou différents,

$R^3$ et $R^{10}$ ont les significations données dans la revendication 1,

X : $(CH_2)_m$ (m = 0, 1, 2, 3, 4), CH=CH, C≡C, $CH_2OCH_2$, $CH_2SCH_2$,

Y: $(CH_2)_m$ (m = 0, 1, 2, 3, 4), O, S, $NR^5$,

Z: $(CH_2)_m$ (m = 0, 1, 2, 3, 4), S, O, un groupe S-alkyle($C_1$-$C_{10}$), (ramifié ou non ramifié), CH=CH, CH=CF, CH=CCl, CH=CBr, $CH_2$-C(O), $CH_2$-CHF, $CH_2$-CHCl, $CH_2$-CHBr, $CH_2$-CHI, cycloalkylène en $C_3$-$C_{10}$, cycloalcénylène en $C_3$-$C_{10}$, $COOR^4$, C≡C, CH=C-alkyle($C_1$-$C_4$) (ramifié ou non ramifié), CH=C(CN), CH=C($R^{10}$), $NR^5$.

**4.** Composés selon la revendication 1, caractérisés en ce que, dans la formule I, D-B-A représente un groupe propylène non substitué ou substitué sur B par deux groupes méthyle.

**5.** Utilisation des composés selon la revendication 1, pour la fabrication de médicaments destinés au traitement du

diabète de type II et d'autres maladies qui sont caractérisées par une production accrue de glucose dans le foie ou une activité accrue du système glucose-6-phosphatase.

6. Médicament contenant un composé selon la revendication 1.